Europäisches Patentamt

(19) European Patent Office

Office européen des brevets

(11) Publication number : **0 498 767 A2**

# (12) EUROPEAN PATENT APPLICATION

(21) Application number : 92810063.5

(22) Date of filing : 28.01.92

(51) Int. Cl.⁵ : **C12N 15/13,** C12P 21/08, C07K 15/28, A61K 39/395, G01N 33/68

The applicant has filed a statement in accordance with Rule 28 (4) EPC (issue of a sample only to an expert). Accession number(s) of the deposit(s): ECACC 91013172.

(30) Priority : 06.02.91 EP 91810086

(43) Date of publication of application :
12.08.92 Bulletin 92/33

(84) Designated Contracting States :
AT BE CH DE DK ES FR GB GR IT LI LU NL PT SE

(71) Applicant : **CIBA-GEIGY AG**
**Klybeckstrasse 141**
**CH-4002 Basel (CH)**

(72) Inventor : **Hardman, Norman, Dr.**
**Gstaltenrainweg 67/3**
**CH-4125 Riehen (CH)**

(54) Chimeric antiidiotypic monoclonal antibodies.

(57) The invention concerns chimeric antiidiotypic monoclonal antibodies consisting of human constant regions and murine variable regions of an antibody which recognizes antibodies directed against HLA class II antigens, and derivatives thereof which retain the specificity of the chimeric antibody from which they are derived. These chimeric antiidiotypic monoclonal antibodies and derivatives have immune-regulatory functions and can therefore be used for diagnostic and therapeutic purposes, such as the treatment of autoimmune diseases.

EP 0 498 767 A2

EP 0 498 767 A2

## Background of the invention

The major histocompatibility complex (MHC), a series of genes coding for protein molecules involved in most reactions of immune recognition in mammalian species, contains three groups of genes: class I and II coding for cell surface recognition molecules, and class III coding for some complement components. In man, the MHC is the HLA (human lymphocyte antigens) gene cluster on chromosome 6. The class II genes are divided into the subregions DP, DQ, DR and DZ. HLA class II antigens, i.e. the proteins encoded by the HLA class II genes, control the immune response by determining recognition interaction of T cells, B cells and macrophages during induction of antibody formation. They are found primarily on B cells and macrophages, including tissue macrophages such as Kupffer cells of the liver, glial cells in the brain, and Langerhans cells in the epidermis. HLA class II antigens consist of two non-covalently associated peptides referred to as $\alpha$ and $\beta$ chains carrying carbohydrate units. The fine structure of each antigen differs with each haplotype. Due to the enormous amount of allotypic variation observed in the HLA region antigens, a large number of MHC specificities are known.

It is known that some diseases are associated with certain HLA specificities, that is to say that individuals with a certain tissue type have a higher risk of developing the disease. In particular, this correlation has been shown for autoimmune diseases, i.e. diseases caused by an immune reaction against self antigens and characterized by the copious production of autoantibodies and autoreactive T cells.

Based on the assumption that in vivo manipulation of the HLA gene function might influence the progression of autoimmune diseases, it has recently been suggested to use specific antiidiotypic antibodies for active immunotherapy, thus taking advantage of idiotype-antiidiotype interactions which are postulated to regulate the immune system. Antiidiotypic antibodies are antibodies directed against specific antibody idiotypes (full set of antibody variable region determinants), which are produced by using antibodies as immunogens. It has been shown that antiidiotypic antibodies raised against primary antibodies directed to a variety of antigens are useful reagents for manipulating the immune response to the corresponding antigens, presumably by balancing amplification and suppressor signals among immune cell subsets. Of special interest for therapeutic application are antiidiotypic antibodies of the internal image type which mimic the initial antigen and in vivo elicit anti-antiidiotypic antibodies which have the same reactivity pattern as the immunizing antibody. For treatment of autoimmune diseases, suitable antiidiotypic antibodies are those which are raised against monoclonal antibodies to HLA antigens.

The development of hybridoma technology made it possible to generate continuous cell lines, in particular murine hybridomas, producing monoclonal antibodies of a desired specificity. However, a major limitation in the use of murine-derived monoclonal antibodies as in vivo diagnostic and therapeutic agents is their immunogenicity as foreign proteins, their rather long persistence in the circulation, and the formation of damaging immune complexes. On the other hand, the treatment with human monoclonal antibodies is also limited since human hybridoma cell lines are hard to prepare, generally unstable, and do not produce monoclonal antibodies of appropriate specificity in sufficient quantities and at reasonable costs.

A promising alternative is the modification of immunoglobulin genes in order to tailor monoclonal antibodies for particular diagnostic and therapeutic tasks. Due to the fact that the variable region and each of the constant region domains of immunoglobulin molecules are encoded in separate exons with their own splice sites, recombinant DNA techniques can be used to isolate different parts of cloned immunoglobulin genes and ligate them to parts of other immunoglobulins. The reconstructed genes are expressed by appropriate transformed continuous cell lines. Murine antibodies can, for example, be converted into "humanized" antibodies by exchanging murine constant region exons for human imunoglobulin constant region exons, thus generating chimeric antibodies withmurine antibody-combining sites and human constant regions. The chimeric antibodies retain the antigen specificity determined by the murine variable regions, but also exhibit human effector functions (such as complement binding, stimulation of phagocytosis, triggering of granule release by mast cells) determined by the carboxy-terminal constant region segments of the heavy chain polypeptides.

An even more sophisticated technique in "humanizing" antibodies described in European Patent Application 0 239 400 exchanges also other fairly conserved regions, the so-called framework regions FRs), within the murine variable regions for corresponding framework regions from human antibodies. Such a humanized antibody should be even less immunogenic in man since the only parts derived from a murine antibody are those hypervariable regions which define a particular specificity for an antigen, the so-called complementarity determining regions (CDRs).

It is believed that by reducing the exposure of the human immune system to the antigenic determinants of the murine antibody domains, especially those in the constant regions of the heavy chains, the possibility of an adverse immunological response will be reduced. This is for example corroborated by Brüggemann et al. (J. Exp. Med. 170, 2153-2157, 1989) who found that about 90% of the immune response is directed against

2

the constant antibody regions. With regard to chimeric antiidiotypic antibodies, in particular those of the internal image type, it is therefore expected that the undesired anti-murine immunoglobulin immune response is reduced, while the idiotypic immune response and thus the production of endogenous anti-antiidiotypic antibodies should not be affected.

## Object of the invention

It is an object of this invention to provide chimeric antiidiotypic monoclonal antibodies (MAbs) consisting of human constant regions and murine variable regions of an antibody which recognizes antibodies directed against HLA class II antigens, for example chimeric antiidiotypic monoclonal antibodies consisting of human constant regions and murine variable regions which are the internal image of determinants recognized by an antibody on HLA class II antigens.

The chimeric antiidiotypic monoclonal antibodies of the invention have immune-regulatory functions and can therefore be used for many diagnostic and therapeutic purposes, such as the treatment of autoimmune diseases.

## Description of the invention

The invention concerns chimeric antiidiotypic monoclonal antibodies (anti-id MAbs) consisting of human constant regions and murine variable regions of an antibody which recognize antibodies (primary antibodies, Abs-1) directed against HLA class II antigens, and derivatives thereof which retain the specificity of the antibody from which they are derived. This means that the chimeric anti-id MAbs of the invention have constant regions which are derived from a human antibody or that the amino acid sequences of the constant regions are homologous to sequences of such a human Ab, and that the chimeric anti-id MAbs of the invention have variable regions which are derived from a murine antiidiotypic antibody recognizing antibodies (Abs-1) directed against HLA class II antigens or that the amino acid sequences of the variable regions are homologous to sequences of such a murine anti-id MAb.

Preferred are chimeric antiidiotypic monoclonal antibodies consisting of human constant regions and murine variable regions which are the internal image of determinants recognized by an antibody (Ab-1) on HLA class II antigens, and derivatives thereof.

Antiidiotypic antibodies are directed against the variable region of another antibody molecule, i.e. against particular antibody idiotypes, and are produced by using antibodies as immunogens. Antiidiotypic antibodies are therefore often designated as Ab-2 (antibody 2) while the immunizing antibody is referred to as Ab-1. Antiidiotypic antibodies of the internal image type are reactive with antigen-binding structures on the immunizing antibody (Ab-1) which are complementary to the antigen, i.e. such antibodies (Abs-2) represent the conformational mirror image of the antigen. Internal image antibodies in vitro inhibit the binding of the immunizing antibody (Ab-1) to target cells, and in vivo elicit anti-antiidiotypic antibodies, also designated Ab-3, which are directed against the antigen and have the same reactivity pattern as Ab-1.

The chimeric anti-id MAbs of the invention are tested for their specificity to the immunizing antibody (Ab-1), for example in an immunoassay such as a binding, crossinhibition or competition radio- or enzyme immunoassay, and for their ability to elicit antibodies (Ab-3) directed against HLA class II antigens, in particular antibodies (Ab-3) which have the same reaction pattern as the immunizing antibody (Ab-1).

The specificity of the chimeric antiidiotypic monoclonal antibodies of the invention determines the type of immune reaction which can be influenced by application of these chimeric anti-id MAbs. This is especially important in the case where certain allospecificities of HLA class II antigens are mimicked by the antiidiotypic MAbs. A number of diseases is known which are associated with particular HLA antigens. For example, there is a correlation between susceptibility to rheumatoid arthritis and expression of HLA-DRw4 antigens, between susceptibility to multiple sclerosis and expression of A3,B7,Bw2,DRw2 antigens, or between susceptibility to myasthenia gravis and expression of B8,DRw3 antigens. Accordingly, for the control and/or treatment of these autoimmune diseases, suitable chimeric anti-id MAbs are those which mimic determinants of the named HLA class II antigen regions.

Particularly preferred are chimeric antiidiotypic monoclonal antibodies consisting of human constant regions and murine variable regions which are the internal image of determinants recognized by an antibody (Ab-1) on the allospecificities DR1,4 of HLA class II antigens, and derivatives thereof.

In particular, the invention concerns chimeric antiidiotypic monoclonal antibodies consisting of human constant regions and murine variable regions which are the internal image of determinants recognized by the monoclonal antibody designated MAb AC 1.59 on the allospecificities DR1,4,w6,w8,w9 of HLA class II antigens, and derivatives thereof. The monoclonal antibody MAb AC1.59 is described by Crepaldi et al. (Tissue Antigens 26, 25, 1985) and recognizes the above-identified polymorphic determinant on HLA-DR antigens.

Especially preferred is the chimeric antiidiotypic monoclonal antibody designated MAb F5-CH$\gamma$1 which is the internal image of determinants recognized by MAb AC1.59 and is capable of eliciting antibodies (Abs-3) which mimic the characteristics of MAb AC1.59, and derivatives thereof, in particular Fab and Fv fragments.

The variable region of an antibody light and heavy chain consists of so-called framework regions (FRs), which are fairly conserved in antibodies with different specificities, and of hypervariable regions also called complementarity determining regions (CDRs), which are typical for a particular specificity.

Preferred chimeric antiidiotypic monoclonal antibodies of the invention and their derivatives are those wherein the light chain variable regions comprise a polypeptide of the formula

$$FR_1\text{-}CDR_{1L}\text{-}FR_2\text{-}CDR_{2L}\text{-}FR_3\text{-}CDR_{3L}\text{-}FR_4\qquad (I)$$

wherein $FR_1$ is a polypeptide residue comprising 19-23 naturally occurring amino acids, $FR_2$ is a polypeptide residue comprising 13-17 naturally occurring amino acids, $FR_3$ is a polypeptide residue comprising 30-34 naturally occurring amino acids, $FR_4$ is a polypeptide residue comprising 7-11 naturally occuring amino acids, $CDR_{1L}$ is a polypeptide residue of the amino acid sequence 22 to 31 of SEQ ID NO:1, $CDR_{2L}$ is a polypeptide residue of the amino acid sequence 47 to 53 of SEQ ID NO:1, and $CDR_{3L}$ is a polypeptide residue of the amino acid sequence 86 to 94 of SEQ ID NO:1, and wherein the amino acid Cys may be in the oxidized state forming S-S-bridges. These particular complementarity determining regions are Ser-Ala-Ser-Ser-Ser-Val-Ser-Tyr-Met-His (CDRiL), Asp-Thr-Ser-Lys-Leu-Thr-Ser (CDR$_{2L}$), and Gln-Gln-Trp-Ser-Ser-Asn-Pro-Leu-Thr (CDR$_{3L}$).

Especially preferred are chimeric monoclonal antibodies and derivatives thereof comprising light chain variable regions of formula I, wherein the polypeptide residues of the framework regions $FR_1$, $FR_2$, $FR_3$ and $FR_4$ are those preferably occurring in mammalian, especially murine or human, antibodies.

Most preferred are chimeric monoclonal antibodies and derivatives thereof according to the invention with light chain variable regions comprising a polypeptide of the amino acid sequence of SEQ ID NO:1, wherein optionally one or more, e.g. 1, 2, 3 or 4, single amino acids within the amino acid sequences 1 to 21 ($FR_1$), 32 to 46 ($FR_2$, 54 to 85 ($FR_3$), and/or 95 to 103 ($FR_4$) are replaced by other amino acids or deleted, and wherein the amino acid Cys may be in the oxidized state forming S-S-bridges, in particular the chimeric monoclonal antibodies and derivatives thereof with light chain variable regions comprising a polypeptide of the amino acid sequence of SEQ ID NO:1, wherein the amino acid Cys may be in the oxidized state forming S-S-bridges. For example, a hydrophobic amino acid within the framework regions may be replaced by another amino acid, preferably also a hydrophobic amino acid, e.g. a homologous amino acid, replaced by two amino acids, or deleted. Likewise, a hydrophilic amino acid within the framework region may be replaced by another amino acid, two amino acids or deleted, whereby replacing amino acids preferably maintain the hydrogen bond structure of the corresponding framework region.

Likewise preferred chimeric antiidiotypic monoclonal antibodies of the invention and their derivatives are those wherein the heavy chain variable regions comprise a polypeptide of the formula

$$FR_5\text{-}CDR_{1H}\text{-}FR_6\text{-}CDR_{2H}\text{-}FR_7\text{-}CDR_{3H}\text{-}FR_8 \qquad (II)$$

wherein $FR_5$ is a polypeptide residue comprising 25-29 naturally occurring amino acids, $FR_6$ is a polypeptide residue comprising 12-16 naturally occurring amino acids, $FR_7$ is a polypeptide residue comprising 30-34 natwally occurring amino acids, $FR_6$ is a polypeptide residue comprising 6-10 naturally occurring amino acids, $CDR_{1H}$ is a polypeptide residue of the amino acid sequence 28 to 32 of SEQ ID NO:2, $CDR_{2H}$ is a polypeptide residue of the amino acid sequence 47 to 62 of SEQ ID NO:2, and $CDR_{3H}$ is a polypeptide residue of the amino acid sequence 95 to 104 of SEQ ID NO:2, and wherein the amino acid Cys may be in the oxidized state forming S-S-bridges. These particular complementarity detemining regions are Asp-Tyr-Val-Val-Ser (CDR$_{1H}$), Val-Ile-Trp-Gly-Gly-Gly-Asn-Thr-Tyr-Tyr-Asn-Ser-Ala-Leu-Lys-Ser (CDR$_{2H}$), and His-Asp-Glu-Ile-Thr-Thr-Tyr-Phe-Asp-Tyr (CDR$_{3H}$).

Especially preferred are chimeric monoclonal antibodies and derivatives thereof comprising heavy chain variable regions of formula II, wherein the polypeptide residues of the framework regions $FR_6$, $FR_6$, $FR_7$ and $FR_6$ are those preferably occurring in mammalian, especially murine or human, antibodies.

Most preferred are chimeric monoclonal antibodies and derivatives thereof according to the invention with heavy chain variable regions comprising a polypeptide of the amino acid sequence of SEQ ID NO:2, wherein optionally one or more, e.g. 1, 2, 3 or 4, single amino acids within the amino acid sequences 1 to 27 ($FR_6$), 33 to 46 ($FR_6$), 63 to 94 ($FR_7$), and/or 105 to 112 ($FR_8$) are replaced by other amino acids or deleted, and wherein the amino acid Cys may be in the oxidized state forming S-S-bridges, in particular the chimeric monoclonal antibodies and derivatives thereof with heavy chain variable regions comprising a polypeptide of the amino acid sequence of SEQ ID NO:2, wherein the amino acid Cys may be in the oxidized state forming S-S-bridges. For example, amino acids within the framework regions may be replaced by other amino acids or deleted as detailed above for the light chain.

Light chain variable regions and heavy chain variable regions may comprise an acyl residue at the N-terminal of SEQ ID NO:1 and SEQ ID NO:2, respectively, for example formyl or alkanoyl, e.g. palmitoyl, myristoyl or lower alkanoyl, such as acetyl or propionyl.

The class of an antibody (immunoglobulin, Ig) molecule is defined by the heavy chain regions. A chimeric monoclonal antibody of the invention may be of any immunoglobulin class, i.e. IgA, IgD, IgE, IgG or IgM. Since different isotypes of antiidiotypic antibodies may have different immune-regulatory action, the antiidiotypic MAbs can be chosen accordingly. A preferential chimeric monoclonal antibody according to the invention is an immunoglobulin of class G which comprises light chain human constant regions $\kappa$ or $\lambda$, especially human constant regions $\kappa$, and heavy chain human constant regions $\gamma$1, $\gamma$2, $\gamma$3 or $\gamma$4, especially human constant regions $\gamma$1.

The invention preferentially concerns a chimeric monoclonal antibody and derivatives thereof with light chain variable regions of formula I with the preferred meaning, e.g. with the amino acid sequence given in SEQ ID NO:1, wherein the amino acid Cys may be in the oxidized state forming S-S-bridges, light chain human constant regions $\kappa$, heavy chain variable regions of formula II with the preferred meaning, e.g. with the amino acid sequence given in SEQ ID NO:2, wherein the amino acid Cys may be in the oxidized state forming S-S-bridges, and heavy chain human constant regions $\gamma$1.

Derivatives of a chimeric antiidiotypic monoclonal antibody of the invention retain the specificity of antibody from which they are derived, i.e. they retain the characteristic binding pattern of the parent antibody. Examples of such derivatives are chimeric antiidiotypic monoclonal antibody fragments, conjugates of the antibody or of a fragment with a compound which enhances the antigenicity, with an enzyme, with a fluorescent marker, with a chemiluminescent marker, with a metal chelate, with avidin, with biotin or the like, or radioactively labelled antibodies or fragments.

Antibody fragments of the invention are for example the univalent fragments Fab or Fab′ or the divalent fragment F(ab′)$_2$. A fragment in a conjugate of the invention may also be a fragment Fv.

Suitable compounds enhancing the antigenicity of the antibodies of the invention are for example lysine rich proteins with free amino groups available for coupling, especially high molecular weight proteins like bovine serum albumin (BSA; MW 66,200), $\alpha$-amylase from <u>Bacillus</u> <u>subtilis</u> (MW 58,000) or keyhole limpet haemocyanin (KLH; MW > 1,000,000) which are commercially available in large quantities. Porcine thyroglobulin, toxins such as tetanus-, cholera- or diphteria-toxins, human serum albumin (HSA), $\beta$-2 microglobulin, and the like, may also be used as compounds enhancing antigenicity. Other possible compounds include polysaccharides, natural or synthetic lipopolysaccharides, synthetic polypeptides such as polylysins, activated membranes, latex particles, bacteria such as <u>Salmonella</u>, and the like. Freund's adjuvant, a water-in-oil emulsion optionally further containing killed mycobacteria, e.g. Bacillus Calmette-Guérin (BCG), may also be used. A preferred compound enhancing antigenicity is a muramyl peptide, in particular the muramyl peptide MTP-PE. Such muramyl peptides and conjugates thereof are disclosed in European Patent Applications 0 003 833 and 0 025 495. Another preferred compound enhancing antigenicity is Alum.

Enzymes used for antibody conjugates of the invention are, for example, horseradish peroxidase, alkaline phosphatase, $\beta$-D-galactosidase, glucose oxidase, glucoamylase, carbonic anhydrase, acetylcholinesterase, lysozyme, malate dehydrogenase or glucose-6-phosphate dehydrogenase.

Fluorescent markers conjugated with antibodies or fragments of the invention can be fluorescein, fluorochrome, rhodamine, and the like.

Chemiluminescence markers are e.g. acridinium esters of luminol.

In such conjugates, the chimeric antibody or fragment is bound to the conjugation partner directly or by way of a spacer or linker group.

Examples of metal chelates are ethylenediaminetetraacetic acid (EDTA), diethylenetriaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like.

Radioactively labelled antibodies or fragments of the invention contain e.g. radioactive iodine ($^{123}$I, $^{125}$I, $^{131}$I), tritium ($^3$H), carbon ($^{14}$C), sulfur ($^{35}$S), yttrium ($^{90}$Y), technetium ($^{99m}$Tc), or the like.

The chimeric antiidiotypic monoclonal antibodies and derivatives thereof according to the invention are prepared by processes that are known <u>per</u> <u>se</u>, characterized in that mammalian cells as defined further below producing such chimeric antiidiotypic monoclonal antibodies are multiplied <u>in</u> <u>vitro</u> or <u>in</u> <u>vivo</u> and, when required, the obtained chimeric antiidiotypic monoclonal antibodies are isolated and/or converted into derivatives thereof.

Multiplication <u>in</u> <u>vitro</u> is carried out in suitable culture media, which are the customary standard culture media, for example Dulbecco's Modified Eagle Medium (DMEM) or RPMI 1640 medium, optionally replenished by a mammalian serum, e.g. fetal calf serum, or trace elements and growth sustaining supplements, e.g feeder cells such as normal mouse peritoneal exudate cells, spleen cells, bone marrow macrophages, 2-aminoethanol, insulin, transferrin, low density lipoprotein, oleic acid, or the like.

<u>In</u> <u>vitro</u> production provides relatively pure antibody preparations and allows scale-up to give large amounts of the desired antibodies. Techniques for mammalian cell cultivation under tissue culture conditions are known in the art and include homogeneous suspension culture, e.g. in an airlift reactor or in a continuous stirrer reactor, or immobilized or entrapped cell culture, e.g. in hollow fibres, microcapsules, on agarose microbeads or ceramic cartridges.

Large quantities of the desired chimeric antiidiotypic monoclonal antibodies can also be obtained by multiplying the cells in vivo. For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl pentadecane), prior to the injection. After one to three weeks, the antibodies are isolated from the body fluids of those mammals. For example, transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The cell culture supernatants are screened for the desired chimeric antiidiotypic monoclonal antibodies, preferentially with an enzyme immunoassay, e.g. a sandwich assay or a dot-assay, or a radioimmunoassay. For example, a sandwich enzyme immunoassay may be used to determine whether correctly assembled immunoglobulins are present in cell culture supernatants, whereby an antibody directed to the light chain human constant region $\kappa$ or $\lambda$ (as appropriate) and another antibody directed to the heavy chain human constant region $\gamma$ of the desired subclass are used, one of which is coated to a solid support and the other one conjugated to an enzyme allowing detection with a suitable enzyme substrate. For the determination whether cell supernatants contain antiidiotypic antibodies of the desired specificity, a sandwich enzyme immunoassay may be used comprising the idiotype (Ab-1), e.g. the monoclonal antibody AC1.59, coated to a solid support and an enzyme conjugated antibody directed to the appropriate light chain or, preferably, heavy chain constant region.

For isolation of the chimeric antiidiotypic monoclonal antibodies, the immunoglobulins in the culture supernatants may be concentrated, e.g. by precipitation with aminonium sulphate, dialysis against hygroscopic material such as PEG, filtration through selective membranes, or the like. If necessary and/or desired, the antibodies are purified by the customary chromatography methods, for example gel filtration, ion-exchange chromatography, chromatography over DEAE-cellulose or (immuno-)affinity chromatography. Preferably, the chimeric antiidiotypic monoclonal antibodies are isolated from cell supernatants containing them by affinity chromatography, for example with Protein A, and/or ion-exchange chromatography.

Fragments of the chimeric antiidiotypic monoclonal antibodies, for example Fab, Fab' or F(ab')$_2$ fragments, can be obtained from the antibodies prepared as described above by methods known per se, e.g. by digestion with enzymes such as papain or pepsin and/or cleavage of disulfide bonds by chemical reduction.

The chimeric antiidiotypic monoclonal antibody derivatives of the invention with enhanced antigenicity are prepared by methods known per se, either by adsorption of an antibody or fragment of the invention to the compound enhancing the antigenicity or by coupling providing chemically bound conjugates. Conjugates of antibodies or fragments of the invention with the mentioned immunogenic compounds or with enzymes are prepared e.g. by reacting an antibody or fragment prepared as described above with the immunogenic compound or the enzyme in the presence of a coupling agent, e.g. glutaraldehyde, periodate, N,N'-o-phenylenedimaleimide, N-(m-maleimidobenzoyloxy)-succinimide, N-(3-[2'-pyridyldithio]-propionoxy)-succinimide, N-ethyl-N'-(3-dimethylaminopropyl)-carbodiimide or the like. Conjugates with biotin are prepared e.g. by reacting antibodies or fragments with an activated ester of biotin such as the biotin N-hydroxysuccinimide ester. Conjugates with fluorescent or chemiluminescent markers are prepared in the presence of a coupling agent, e.g. those listed above, or by reaction with an isothiocyanate, preferably fluorescein-isothiocyanate.

Chimeric antiidiotypic monoclonal antibodies or fragments radioactively labelled with iodine ($^{123}$I, $^{125}$I, $^{131}$I) are obtained from the antibodies or fragments of the invention by iodination known per se, for example with radioactive sodium or potassium iodide and a chemical oxidizing agent, such as sodium hypochlorite, chloramine T or the like, or an enzymatic oxidizing agent, such as lactoperoxidase, or glucose oxidase and glucose. Antibodies or fragments according to the invention are coupled to yttrium ($^{90}$Y) for example by diethylenetriaminepentaacetic acid (DPTA)-chelation. Technetium-99m labelled antibodies or fragments are prepared by ligand exchange processes, for example by reducing pertechnate (TcO$_4^-$) with stannous ion solution, chelating the reduced technetium onto a Sephadex column and applying the antibodies or fragments to this column, or by direct labelling techniques, e.g. by incubating pertechnate, a reducing agent such as SnCl$_2$, a buffer solution such as sodium-potassium phthalate solution, and the antibodies or fragments.

Fragments and conjugates of antibodies or fragments with compounds enhancing antigenicity or enzymes may also be prepared directly by recombinant DNA techniques as will be described below.

The invention also concerns recombinant DNAs comprising an insert coding for a light chain murine variable region and/or for a heavy chain murine variable region of antiidiotypic antibodies which recognize antibodies (Abs-1) directed against HLA class II antigens as described hereinbefore. By definition such DNAs comprise coding single stranded DNAs, double stranded DNAs consisting of said coding DNAs and of complementary DNAs thereto, or these complementary (single stranded) DNAs themselves.

Particularly preferred are recombinant DNAs comprising an insert coding for a light chain murine variable region and/or for a heavy chain murine variable region of antiidiotypic antibodies which are the internal image of determinants recognized by an antibody (Ab-1) on the allospecificities DR1,4 of HLA class II antigens.

In particular the invention concerns a recombinant DNA comprising an insert coding for a light chain murine variable region, which originates from genomic DNA of the hybridoma cell line F5-444, or which is homologous to genomic DNA of said cell line and codes for an amino acid sequence homologous to the light chain variable region of monoclonal antibody F5-444. The hybridoma cell line F5-444 was generated by fusion of a mouse myeloma cell and a B lymphocyte of a Balb/c mouse which had been immunized with MAb AC1.59 (F. Perosa and S. Ferrone, J. Clin. Invest. $\underline{84}$, 907-914, 1989). The cell line F5-444 produces the murine antiidiotypic monoclonal antibody MAb F5-444 which is the internal image of determinants recognized by MAb AC1.59.

Preferred is a recombinant DNA comprising an insert coding for the polypeptide of formula I, wherein $FR_1$, $FR_2$, $FR_3$, $FR_4$, $CDR_{1L}$, $CDR_{2L}$, and $CDR_{3L}$ have the meanings as mentioned hereinbefore, optionally further containing introns. Especially preferred is a recombinant DNA coding for the polypeptide of formula I comprising inserts coding for murine or human timework regions $FR_1$, $FR_2$, $FR_3$ and $FR_4$, and inserts coding for complementarity determining regions of the DNA sequence 70 to 99 ($CDR_{1L}$, the DNA sequence 145 to 165 ($CDR_{2L}$, and the DNA sequence 262 to 288 ($CDR_{3L}$ of SEQ ID NO:1. Most preferred is a DNA comprising an insert of the DNA sequence 7 to 316 of SEQ ID NO:1, wherein optionally one or more, e.g. 1 to 10, nucleotides are replaced by other nucleotides, in particular a DNA comprising an insert of the DNA sequence 7 to 316 of SEQ ID NO:1. In a DNA wherein nucleotides of the sequence given in SEQ ID NO: 1 are replaced by other nucleotides, such replacement is preferred when it does not alter the amino acid sequence of the complementarity determining regions (CDRs) coded for. This means that such replacement of nucleotides may occur in the inserts coding for the framework regions (FRs) or in a position where it does not alter the amino acid coded for due to the degeneracy of the triplet codons.

Likewise the invention concerns a recombinant DNA comprising an insert coding for a heavy chain murine variable region, which originates from genomic DNA of the hybridoma cell line F5-444, or which is homologous to genomic DNA of said cell line and codes for an amino acid sequence homologous to the heavy chain variable region of monoclonal antibody F5-444.

Preferred is a recombinant DNA comprising an insert coding for the polypeptide of formula II, wherein $FR_5$, $FR_6$, $FR_7$, $FR_6$, $CDR_{1H}$, $CDR_{2H}$, and $CDR_{3H}$ have the meanings as mentioned hereinbefore, optionally further containing introns. Especially preferred is a recombinant DNA coding for the polypeptide of formula II comprising inserts coding for murine or human framework regions $FR_5$, $FR_6$, $FR_7$ and $FR_8$, and inserts coding for complementarity determining regions of the DNA sequence 90 to 104 ($CDR_{1H}$), the DNA sequence 147 to 194 ($CDR_{2H}$), and the DNA sequence 291 to 320 ($CDR_{3H}$) of SEQ ID NO:2. Most preferred is a DNA comprising an insert of the DNA sequence 9 to 345 of SEQ ID NO:2, wherein optionally one or more, e.g. 1 to 10, nucleotides are replaced by other nucleotides, in particular a DNA comprising an insert of the DNA sequence 9 to 345 of SEQ ID NO:2. In a DNA wherein nucleotides of the sequence given in SEQ ID NO:2 are replaced by other nucleotides, such replacement is preferred when it does not alter the amino acid sequence of the complementarity determining regions (CDRs) coded for, as is described above for DNA coding for the light chain variable region.

For the assembly of complete tetrameric immunoglobulin molecules and the expression of active antibodies, the recombinant DNA inserts coding for light and heavy chain variable regions are fused with the corresponding DNAs coding for light and heavy chain constant regions, then transferred into appropriate host cells, for example after incorporation into hybrid vectors.

The invention therefore also concerns recombinant DNAs comprising an insert coding for a light chain murine variable region of an antibody, which recognizes antibodies directed against HLA class II antigens, fused to a human constant region $\kappa$ or $\lambda$. Preferred is a recombinant DNA coding for a preferred murine variable region as described hereinbefore fused to a human constant region $\kappa$.

Likewise the invention concerns recombinant DNAs comprising an insert coding for a heavy chain murine variable region of an antibody, which recognizes antibodies directed against HLA class II antigens, fused to a human constant region $\gamma$, for example $\gamma1$, $\gamma2$, $\gamma3$ or $\gamma4$. Preferred is a recombinant DNA coding for a preferred murine variable region as described hereinbefore fused to a human constant region $\gamma1$.

The invention further concerns recombinant DNAs coding for fragments of chimeric antiidiotypic monoclonal antibodies as defined hereinbefore, e.g. DNA coding for Fab, Fab' or Fv fragments of such antibodies, and for conjugates of antibodies or fragments as defined hereinbefore, e.g. for fusion proteins comprising a light or heavy chain of such antibody fused to a compound enhancing antigenicity as defined hereinbefore or fused to an enzyme.

Furthermore the invention concerns a recombinant DNA which is a hybrid vector comprising an insert coding for a chimeric murine/human light chain as described hereinbefore and/or an insert coding for a chimeric murine/human heavy chain as described hereinbefore, an origin of replication or an autonomously replicating sequence, one or more dominant marker sequences and, optionally, expression control sequences, signal sequences and additional restriction sites.

Vectors typically perform two functions in collaboration with compatible host cells. One function is to facilitate the cloning of the nucleic acid that encodes the chimeric immunoglobulin chains, i.e. to produce usable quantities of the nucleic acid (cloning vectors). The other function is to provide for replication and expression of the chimeric gene constructs in a suitable host, either by maintenance as an extrachromosomal element or by integration into the host chromosome (expression vectors). A cloning vector comprises the chimeric gene constructs as described above, an origin of replication or an autonomously replicating sequence, dominant marker sequences and, optionally, signal sequences and additional restriction sites. An expression vector additionally comprises expression control sequences essential for the transcription and translation of the chimeric genes.

An origin of replication or an autonomously replicating sequence is provided either by construction of the vector to include an exogeneous origin such as derived from Simian virus 40 (SV 40) or another viral source, or by the host cell chromosomal mechanisms.

The markers allow for selection of host cells which contain the vector. Selection markers include genes which confer resistance to heavy metals such as copper or to antibiotics such as tetracycline, ampicillin, geneticin (G-418), neomycin, kanamycin or hygromycin, or genes which complement a genetic lesion of the host cell such as the absence of thymidin kinase, hypoxanthine phosphoryl transferase, dihydrofolate reductase or the like.

Signal sequences may be, for example, presequences or secretory leaders directing the secretion of the antibody, splice signals, or the like.

As expression control sequences, the vector DNA comprises a promoter, sequences necessary for the initiation and termination of transcription and for stabilizing the mRNA and, optionally, enhancers and further regulatory sequences. A wide variety of promoting sequences may be employed, depending on the nature of the host cell. Promoters suitable for mammalian host cells are obtained from viruses such as Simian virus 40 (SV 40), Rous sarcoma virus (RSV), adenovirus 2, bovine papilloma virus (BPV), papovavirus BK mutant (BKV), or mouse or human cytomegalovirus (CMV). Alternatively, the vectors may comprise promoters from mammalian expression products, such as actin, collagen, myosin etc., or the native promoter and control sequences which are normally associated with the immunoglobulin gene sequences. Sequences necessary for the initiation and termination of transcription and for stabilizing the mRNA are commonly available from the noncoding 5′ regions and 3′ regions, respectively, of viral and eukaryotic cDNAs, e.g. from the expression host. Enhancers are transcription-stimulating DNA sequences of viral origin, e.g. derived from Simian virus, polyoma virus, bovine papilloma virus or Moloney sarcoma virus, or of genomic, especially murine, origin.

The various DNA segments of the vector DNA are operationally linked, i.e. they are contiguous and placed into a functional relationship with each other.

Preferred vectors are suitable for mammalian hosts and are based on viral replication systems. Particularly preferred are vectors comprising Simian virus promoters, e.g. pSVgpt or pSVneo, further comprising an enhancer, e.g. an enhancer normally associated with the immunoglobulin gene sequences, in particular the mouse Ig H or L chain enhancer.

The chimeric gene constructs for the light chain and for the heavy chain are sequentially or simultaneously transferred into the host cells with the help of two vectors. Alternatively, both heavy and light chains are cloned into the same hybrid vector and incorporated in a one step-procedure as a single construct into the host cells. A third alternative utilises co-transfection of unlinked DNA fragments.

The recombinant DNAs coding for the desired chimeric antiidiotypic monoclonal antibodies can be prepared, for example, by culturing a transformed host cell.

In particular, such DNAs can be prepared by

a) isolating murine DNAs from a suitable hybridoma cell line and selecting the desired DNAs coding for the viable regions of antiidiotypic antibodies with the desired specificity using DNA probes,

b) isolating human DNAs from a genomic library and selecting the desired DNAs coding for the constant regions of antibodies using DNA probes,

c) constructing chimeric mouse/human genes by incorporating the DNA of step a) and b) into appropriate hybrid vectors,

d) transferring the obtained hybrid vectors into a recipient host cell or retrieving the DNA coding for the chimeric mouse/human genes and transferring the unlinked DNA into a recipient host cell,

e) selecting and culturing the transformed host cell, and

f) optionally isolating the desired DNA.

The DNA according to step a) of the process described above can be obtained by isolation of genomic DNA or by preparation of cDNA from isolated mRNA. Genomic DNA from hybridoma cells is isolated by methods known in the art which include steps for disruption of the cells, e.g. by lysis in presence of detergents like Triton™, extracting the DNA, e.g. by treatment with phenol and $CHCl_3$/isoamyl alcohol, and precipitation of DNA. The DNA is fragmented, conveniently by one or more restriction endonucleases, e.g. XbaI, BglII, EcoRI, HindIII, BamHI, the resulting fragments are replicated on a suitable carrier, e.g. nitrocellulose membranes, and screened with a DNA probe as described in more detail hereinbelow for the presence of the DNA sequences coding for the polypeptide sequence of interest, in particular for the presence of the rearranged H- and L-chain Ig gene loci. By this procedure DNA fragments are found that contain inserts with heavy chain V, D and J regions and light chain V and J regions, respectively, together with a leader sequence and introns, if any. cDNA from hybridoma cells is likewise prepared by methods known in the art, e.g. by extracting total cellular RNA, isolating mRNA by a suitable chromatographic method, e.g. chromatography on oligo(dT)-cellulose, synthesizing cDNA with a mixture of deoxynucleotide triphosphate and reverse transcriptase in the presence of oligonucleotide primers complementary to suitable regions in the murine immunoglobulin heavy and light chain constant genes, and isolating the cDNA. As a tool simplifying DNA isolation, the desired genomic DNA or cDNA may be amplified using polymerase chain reaction (PCR) technology. PCR involves repeated rounds of extension from two primers specific for DNA regions at each end of the gene. Preferably, cDNA transcripts of total mRNA from the suitable hybridoma cell line is treated in a heating/cooling cycle with Taq DNA polymerase in the presence of primers tailored to hybridize to Ig H and L chain variable regions, respectively.

Genomic human DNA according to step b) of the process described above is isolated from suitable human tissue, preferably from human placenta or human foetal liver cells, according to methods known in the art. A genomic DNA library is constructed therefrom by limited digestion with suitable restriction endonucleases, e.g. HaeIII and AluI, and incorporation into λ Charon phage, e.g. λ Charon 4a, following established procedures. The genomic DNA library is replicated, e.g. on nitrocellulose membranes, and screened with a DNA probe as described below for the DNA sequences of interest. The desired DNA may be amplified using PCR technology.

The DNA probe for the mouse variable regions or the human constant regions may be a synthetic DNA, a cDNA derived from mRNA coding for the desired immunoglobulin or a genomic DNA or DNA fragment of known nucleotide sequence. As probes for the detection and/or amplification of the rearranged Ig gene loci of the variable regions of L-/H-chains, DNA fragments of known nucleotide sequences of adjacent conserved variable or constant regions are selected which constitute the Ig loci of the L-/H-chain in the mammal from which the DNA is derived, e.g. Balb/c mice. The possible utilization of murine DNA probes for the detection of human DNA sequences is based on sequence homologies between the murine and human DNAs. The DNA probe is synthesized or isolated from suitable tissue of an appropriate mammal, e.g. Balb/c mouse liver, and purified by standard methods. If required, the probe DNA is labelled, e.g. radioactively labelled by the well-known nick-translation technique, then hybridized with the human DNA library in buffer and salt solutions containing adjuncts, e.g. calcium chelators, viscosity regulating compounds, proteins, non-specific DNA and the like, at temperatures favoring selective hybridization.

Once a fragment has been identified which contains the desired DNA sequence, this fragment may be further manipulated to remove nonessential DNA, modified at one or both termini, and treated to remove all or a portion of intervening sequences, or the like.

The joining of the various DNA fragments in order to produce chimeric genes is performed in accordance with conventional techniques, for example, by blunt- or staggered-end ligation, restriction enzyme digestion to provide for appropriate cohesive termini, filling in cohesive ends as appropriate, alkaline phosphatase treatment to avoid undesirable joining, and ligation with appropriate ligases.

The transfer of the recombinant DNAs, e.g. the transfer of hybrid vectors, and the selection of transformed cells is described below.

Moreover, the invention relates to host cells transformed with the recombinant DNAs described above, namely host cells which are transformed with a DNA encoding the light chain and/or a DNA encoding the heavy chain of the desired chimeric antiidiotypic monoclonal antibody.

The host cells of the present invention have to be capable of culture in vitro and have to be of higher eukaryotic origin to provide a suitable environment for the production of active antibodies, since the biosynthesis of functional tetrameric antibody molecules requires correct nascent polypeptide chain folding, glycosylation, and assembly.

Examples of suitable host cells according to the invention are mammalian cells, e.g. COS-7 cells, Bowes melanoma cells, chinese hamster ovary (CHO) cells, embryonic lung cells L-132, and in particular mammalian cells of lymphoid origin, such as lymphoma, myeloma, hybridoma, trioma or quadroma cells, for example PAI, Sp2/0 or X63-Ag8.653 cells. Preferred are cells of the cell line Sp2/0, which is a well-characterized, Ig non-secreting mouse cell line derived from the fusion of mouse spleen cells with the myeloma X63-Ag8.

These host cells are transfected with the chimeric L-chain gene construct alone, with the chimeric H-chain gene construct alone, or with both, either sequentially or simultaneously transferred with the help of two separate vectors or in a one-step procedure by using a double-construct (L-chain/H-chain) vector as indicated hereinbefore. In the alternative, unlinked chimeric gene constructs may be transfected into the host cells either sequentially or simultaneously.

Preferred are host cells transfected with both gene constructs secreting chimeric antiidiotypic monoclonal antibodies which recognize monoclonal antibodies directed against HLA class II antigens as described hereinbefore, for example cells of the cell line F5-CHγ1. Clone D4 of the preferred cell line F5-CHγ1 has been deposited for patent purposes at the European Collection of Animal Cell Cultures (ECACC), Porton Down, Salisbury, U.K., on January 31, 1991 under the accession number ECACC 91013172. Further examples of host cells of the invention are cells transfected with similar recombinant plasmids which contain alternative orientations of the H- and L-chain gene constructs, incorporating additional DNA elements to facilitate high levels of expression of the chimeric monoclonal antibodies.

The host cells of the invention are genetically stable, secrete chimeric antiidiotypic monoclonal antibodies of the invention of constant specificity and can be activated from deep-frozen cultures by thawing and recloning.

The invention also relates to processes for the preparation of host cells secreting chimeric antiidiotypic monoclonal antibodies which recognize antibodies directed against HLA class II antigens as described hereinbefore, characterized in that a suitable cell is transformed with one or two vectors as described hereinbefore.

Vectors or unlinked DNAs are introduced into the host cells by conventional techniques, such as calcium phosphate precipitation, microinjection into the cell nucleus, protoplast fusion, electroporation, i.e. introduction of DNA by a short electrical pulse which transiently increases the permeability of the cell membrane, or the like. Transfection may be carried out in the presence of helper compounds, e.g. diethylaminoethyldextran, dimethyl sulfoxide, glycerol, polyethylene glycol or the like, or as co-precipitates of vector DNA and calcium phosphate.

After the transfection procedure, transfected cells are identified and selected with the help of a selection procedure matching the selection marker of the DNA used for transfection. Selection markers include genes which confer resistance to heavy metals such as copper or to antibiotics, e.g. G-418 (geneticin, a neomycin-derivative) or hygromycin, or genes which complement a genetic lesion of the host cell such as the absence of thymidine kinase, hypoxanthine phosphoribosyl transferase, dihydrofolate reductase, or the like. For example, if the DNA used for transfection comprises a marker for geneticin resistance, transformed cells are identified and separated from untransformed cells by culture in the presence of the antibiotic geneticin.

The chimeric antiidiotypic monoclonal antibodies and their derivatives according to the invention are useful for a number of therapeutic and diagnostic purposes. In particular, the invention concerns a method of treating autoimmune diseases and allograft rejections by modulation of the immune response to HLA class II antigens.

The chimeric antiidiotypic monoclonal antibodies and their derivatives according to the invention can, for example, be used to modulate the immune response to HLA class II antigens due to their immune-regulatory functions within the idiotype-antiidiotype reaction network. The modulation is specific and is determined by the choice of the antibody (Ab-1) used for the production of the murine antiidiotypic MAbs from which the variable regions are derived, i.e. the specificity of the immunizing Ab-1, and by the isotype of the antibody of the invention, since different isotypes of antiidiotypic antibodies have different immune-regulatory functions. The chance of an adverse immunological response to the antibody of the invention as such is considerably reduced in comparison to adverse reactions caused by repeated application of comparable murine or other non-human antibodies. The antibodies of the invention which are of the internal image type are capable of eliciting antibodies (Abs-3) directed against HLA class II anti antigens. These elicited antibodies have the same general reactivity pattern as the antibody (Ab-1) originally used for immunization. The stimulation of endogenous production of antibodies (Abs-3) directed against HLA class II antigens overcomes the drawbacks of direct application of such immunizing antibodies (Abs-1) in a passive immunotherapy, i.e. the necessity of a great number of injections and large doses. Consequently, the chimeric antiidiotypic monoclonal antibodies and derivatives thereof according to the invention, in particular fragments and conjugates with compounds enhancing antigenicity, are useful agents for the control and treatment of autoimmune diseases.

Autoimmune diseases which can be treated by this method are for example rheumathoid arthritis (RA), systemic lupus erythematosus (SLE), progressive systemic sclerosis (scleroderma), rheumatic fever, polyarthritis nodosa, Sjögren's syndrome, thrombotic thrombocytopenic purpura and other connective tissue disorders, multiple sclerosis (MS), insulin-dependent diabetes mellitus (IDDM), myasthenia gravis and the like, in particular rheumatoid arthritis. Since in many cases the association with particular HLA antigens is known in such diseases, the antiidiotypic MAbs of the invention can be "tailor-made" to mimic the concerned antigens by the adequate choice of the immunizing antibody (Ab-1). Furthermore, the chimeric antiidiotypic monoclonal antibodies and/or derivatives thereof according to the invention can be used to prevent and control allograft rejection by suppressing the immune response to the mismatched HLA antigens.

The invention also concerns pharmaceutical compositions for treating autoimmune diseases and allograft rejections by modulation of the immune response to HLA class II antigens comprising a therapeutically effective amount of a chimeric antiidiotypic monoclonal antibody and/or of a derivative thereof according to the invention and a pharmaceutically accetable carrier. Derivatives of antibodies that are considered are fragments or conjugates with compounds enhancing antigenicity.

Preferred are pharmaceutical compositions for parenteral application and inhalation, e.g. nasal application. Compositions for intramuscular, subcutaneous or intravenous application or for inhalation are e.g. isotonic aqueous solutions or suspensions, optionally prepared shortly before use from lyophilized or concentrated preparations. Suspensions in oil contain as oily component the vegetable, synthetic or semi-synthetic oils customary for injection purposes. The pharmaceutical compositions may be sterilized and contain adjuncts, e.g. for conserving, stabilizing, wetting, emulsifying or solubilizing the ingredients, salts for the regulation of the osmotic pressure, buffer and/or compounds regulating the viscosity, e.g. sodium carboxycellulose, carboxymethylcellulose, sodium carboxymethylcellulose, dextran, polyvinylpyrrolidine or gelatine.

Preferred are pharmaceutical compositions further comprising adjuvants which positively influence the formation of endogenous antibodies (Abs-3). Adjuvants considered are complete Freund's adjuvant (emulsion of mineral oil, water, and mycobacterial extracts), incomplete Freund's adjuvant (emulsion of water and oil only), mineral gels, e.g. aluminium hydroxide gels, surface active substances such as lysolecithin, polyanions, peptides, BCG (Bacillus Calmette-Guérin), or the compounds enhancing antigenicity mentioned above. Particularly preferred are the muramyl peptide MTP-PE or Alum. If the pharmaceutical composition contains conjugates of antibodies or fragments of the invention with compounds enhancing antigenicity, no further adjuvants are required.

The pharmaceutical compositions of the invention contain from approximately 0.01% to approximately 50% of active ingredients. They may be in dosage unit form, such as ready-to-use ampoules or vials, or also in lyophylized solid form.

In general, the therapeutically effective dose for mammals is between approximately 5 and 25 µg of chimeric antiidiotypic monoclonal antibodies of the invention and/or derivatives thereof per kg body weight depending on the status of the patient and the mode of application. The specific mode of administration and the appropriate dosage will be selected by the attending physician taking into account the particulars of the patient, the state of the disease, the type of autoimmune disease or immunological disorder treated, and the like.

The pharmaceutical compositions of the invention are prepared by methods known in the art, e.g. by conventional mixing, dissolving, confectioning or lyophilizing processes. Pharmaceutical compositions for injection are processed, filled into ampoules or vials, and sealed under aseptic conditions according to methods known in the art.

The pharmaceutical compositions may also be for enteral, such as rectal or oral, administration. Pharmaceutical compositions for oral application can be obtained by combining the active ingredient with solid carriers, optionally granulating a resulting mixture and, if desired or necessary after the addition of suitable adjuncts, processing the mixture or granulate into tablets or dragée cores. They can also be incorporated into plastics carriers which release the active ingredients, or allow them to diffuse, in a controlled manner.

The chimeric antiidiotypic MAbs and derivatives thereof according to the invention can also be used for the qualitative and quantitative determination of antibodies directed against HLA class II antigens. This is especially useful for the monitoring of allograft rejection, for the diagnosis of autoimmune diseases, for the decision whether an autoimmune disease is amenable to treatment with the chimeric antiidiotypic monoclonal antibodies of the invention, and for monitoring the treatment of autoimmune diseases with the chimeric antiidiotypic MAbs and derivatives thereof.

In general, the chimeric antiidiotypic monoclonal antibodies or derivatives thereof according to the invention can be used in any of the known immunoassays which rely on the binding interaction between the idiotopes of the antibodies directed against HLA class II antigens and of the antiidiotypic antibodies. Examples of such assays are radio-, enzyme, fluorescence, chemiluminescence, immunoprecipitation, latex agglutination, and hemagglutination immunoassays.

The chimeric antiidiotypic monoclonal antibodies according to the invention can be used as such or in the form of radioactively labelled derivatives in a radioimmunoassay (RIA). Any of the known modifications of a RIA can be used, for example soluble phase (homogeneous) RIA, solid phase (heterogeneous) RIA, single RIA or double (sandwich) RIA with direct or indirect (competitive) determination of antibodies directed against HLA class II antigens.

An example of such a radioimmunoassay is a sandwich RIA in which a suitable carrier, for example the plastic surface of a microtiter plate or of a test tube, e.g. of polystyrene, polypropylene or polyvinylchloride, glass or plastic beads, filter paper, dextran etc. cellulose acetate or nitrocellulose sheets, magnetic particles or the like, is coated with a chimeric antiidiotypic monoclonal antibody of the invention by simple adsorption or optionally after activation of the carrier, for example with glutaraldehyde or cyanogen bromide. Then test solutions containing antibodies directed against HLA class II antigens and finally polyclonal antibodies which also react with the anti-HLA antibodies and which are radioactively labelled, e.g. with $^{125}$I, are added. The amount of antibodies directed against HLA class II antigens in the test solution is directly proportional to the amount of bound polyclonal antibodies and is determined by measuring the radioactivity of the solid phase.

The chimeric antiidiotypic monoclonal antibodies according to the invention can be used as such or in the form of enzyme-conjugated derivatives in an enzyme immunoassay. As described above for radioimmunoassays, any of the known modifications of an enzyme immunoassay can be used.

The tests are carried out in an analogous manner to the radioimmunoassays described above using an enzyme label instead of a radioactive label. The amount of immune complex formed which corresponds to the amount of antibodies directed against HLA class II antigens present in the test solutions is determined by adding an enzyme substrate solution. The enzyme substrate reaction results, for example, in a color change which can be observed by eye or with optical measuring devices.

The chimeric antiidiotypic monoclonal antibodies according to the invention can be used as such or in the form of derivatives conjugated with chemiluminescent markers in a chemiluminescence immunoassay. As described above for radioimmunoassays, any of the known modifications of a chemiluminescence immunoassay can be used.

The tests are carried out in an analogous manner to the radioimmunoassays described above using a chemiluminescent label instead of a radioactive label. The amount of immune complex formed which corresponds to the amount of antibodies directed against HLA class II antigens present in the test solutions is determined by adding a compound triggering luminescence, e.g. $H_2O_2$ and NaOH, and measuring the emission of light with optical measuring devices.

The use according to the invention of antiidiotypic monoclonal antibodies and derivatives thereof as described hereinbefore for the determination of antibodies directed against HLA class II antigens also includes other immunoassays known per se, for example immunofluorescence assays, latex agglutination, hemagglutination, evanescent light assays using an optical fibre coated with an antiidiotypic MAb and other direct-acting immunosensors which convert the binding event into an electrical or optical signal, or the like.

The invention also concerns test kits for the qualitative and quantitative determination of antibodies directed against HLA class II antigens comprising chimeric antiidiotypic monoclonal antibodies of the invention and/or derivatives thereof and, optionally, other polyclonal or monoclonal antibodies and/or adjuncts.

Test kits according to the invention for a radioimmunoassay contain, for example, a suitable carrier, optionally freeze-dried solutions of one or more polyclonal and/or monoclonal antibodies, solutions of a radioactively labelled antibody, standard solutions of antibodies directed against HLA class II antigens, buffer solutions, and, optionally, polypeptides or detergents for preventing non-specific adsorption and aggregate formation, pipettes, reaction vessels, calibration curves, instruction manuals and the like. One of the antibodies of the test kit is an antiidiotypic monoclonal antibody of the invention.

Test kits according to the invention for an enzyme immunoassay contain, for example, a suitable carrier, optionally freeze-dried solutions of one or more polyclonal and/or monoclonal antibodies, optionally freeze-dried or concentrated solutions of an enzyme- or biotin-conjugated antibody, solutions of an enzyme-avidin conjugate if biotin-labelled antibody is used, enzyme substrate in solid or dissolved form, standard solutions of antibodies directed against HLA class II antigens, buffer solutions, and, optionally, polypeptides or detergents for preventing non-specific adsorption and aggregate formation, pipettes, reaction vessels, calibration curves, instruction manuals and the like. One of the antibodies of the test kit is an antiidiotypic monoclonal antibody of the invention.

The following examples illustrate the invention but do not limit it to any extent.

Abbreviations

| ATP | adenosine triphosphate |
|---|---|
| bp | base pair |
| BSA | bovine serum albumin |
| CDR | complementarity determining region |
| CIAP | calf intestinal alkaline phosphatase |
| CIAP-buffer (10x) | 10 mM $ZnCl_2$, 10 mM $MgCl_2$, 0.5 M Tris-HCl, pH 7.6 |
| DNA ligase buffer(10x) | 0.1 M $MgCl_2$, 0.5 M Tris-HCl, pH 7.6 |
| dNTP | deoxyribonucleotide triphosphate |

| | |
|---|---|
| DTT | dithiothreitol |
| EDTA | ethylenediaminetetraacetic acid |
| FCS | fetal calf serum |
| H-chain | heavy chain |
| HEPES | N-2-hydroxyethyl-piperazine-N'-2-ethanesulfonic acid |
| HT | hypoxanthine/thymidine |
| L-chain | light chain |
| Ig | immunoglobulin |
| kb | kilobase pairs |
| MAb | monoclonal antibody(ies) |
| NT-buffer (10x) | 0.1 M $MgCl_2$, 1 mM dithiothreitol, 500 µg BSA/ml (Serva, Fraction V), 0.5 M Tris-HCl, pH 7.5 |
| PBS | phosphate buffered saline solution (Dulbecco & Vogt, J. Exp. Med. 99, 167, 1954) |
| PBS-CM | PBS without $MgCl_2$ and $CaCl_2$ |
| PCR-buffer (10x) | 15 mM $MgCl_2$, 0.5 M KCl, 0.1 M β-mercaptoethanol, 0.5% (w/v) Tween-20™ (Merck), 0.5% (w/v) NP-40™ (Merck), 0.1 M Tris-HCl, pH 8. 3 |
| PNK-buffer | 0.1 M $MgCl_2$, 1 mM EDTA, 0.5 M Tris-HCl, pH 7.5 |
| RIA-buffer | PBS-buffer containing 1% BSA (Serva, Fraction V), 0.2% $NaN_3$ (Merck), 0.05% (w/v) Tween-20% (Merck) |
| RT-buffer (5x) | 15 mM $MgCl_2$, 0.375 M KCl, 50 mM DTT, 0.25 M Tris-HCl, pH 8.3 |
| Substrate buffer | 10% (v/v) diethanolamine (Merck), 0.2% $NaN_3$ (Merck), 0.1% $MgCl_2$, pH adjusted to 9.8 with HCl |
| TAE-buffer | 1 mM EDTA, 0.04 M Tris-acetate |
| TBE-buffer | 89 mM boric acid, 2 mM EDTA, 89 mM Tris-borate |
| TE-buffer | 1 mM EDTA, 10 mM Tris-HCl, pH 8.0 |
| Tris | Tris(hydroxymethyl)aminomethane |
| V-region | variable region |

Examples

Example 1. <u>Cloning and adaptation of functional Ig H- and L-chain V-region exons of murine hybridoma F5-444 for expression in Sp2/0 myeloma cells</u>

General methods used are described in detail in Sambrook et al. (Molecular Cloning: A Laboratory Manual, Second Edition, Cold Spring Harbor Press, 1989).

1.1 <u>Origin of murine hybridoma F5-444 and preparation of RNA:</u>

The manufacture and properties of the murine hybridoma designated F5-444 is described in F. Perosa & S. Ferrone, "Murine antiidiotypic monoclonal antibodies that bear the internal image of HLA-DR allospecificities", J. Clin. Invest. <u>84</u>, 907 (1989). It is prepared from B lymphocytes raised in Balb/c mice against idiotypic determinants on the murine MAb AC 1.59, and represents an internal image of determinants recognized by this anti-DR1,4,w6,w8,w9 MAb. Total RNA is isolated from ca. $0.5\text{-}1x10^8$ cells using the procedure described by Le Meur et al. (Cell <u>23</u>, 561-571, 1981).

1.2 <u>In vitro amplification of functional H- and L-chain Ig cDNA sequences:</u>

Nucleotide sequences corresponding to the coding regions of Ig genes expressed in the hybridoma F5-444 are obtained by reverse transcription of total hybridoma RNA, and amplification <u>in</u> <u>vitro</u> of the Ig cDNA transcripts using Taq DNA polymerase. The following oligonucleotide primers are used (letters in brackets signify degenerate nucleotides at these positions):

VH1FOR: 5'-TGAGGAGACGGTGACCGTGGTCCCTTGGCCCCAG-3'

VH1BACK: 5'-AGGT(G/C)(C/A)A(G/A)CTGCAG(G/C)AGTC(T/A)GG-3'

VK1FOR: 5'-GTTAGATCTCCAGCTTGGT(G/C)C(G/C)-3'

VK1BACK: 5'-GACATTCAGCTGACCCAGTCTCCA-3'

M/Cκ: 5'-GGGAAGATGGATACAGTTGG-3'

M/Cκ corresponds to the reverse antisense strand of the mouse Ig L-chain Cκ constant region exon (Hieter et al., Cell 22, 197-207, 1980). VH1FOR, VH1BACK, VK1FOR and VK1BACK correspond to the Ig H- and L-chain V-region primers described by Orlando et al. (Proc. Natl. Acad. Sci. USA 86, 3833-3837, 1989), with the exception that VK1FOR used here includes two degenerate base substitutions (underlined above). The latter set of primers include DNA restriction sites which facilitate later cloning of amplified V-regions (see example 1.3). These are: VH1FOR: BstEll (GGTGACC); VH1BACK: PstI (CTGCAG); VK1FOR: Bglll (AGATCT); VK1BACK: PvuII (CAGCTG).

For reverse transcription and amplification of Ig H-chain mRNA, total F5-444 hybridoma RNA (10 μg) is treated for 90 min at 37°C with 200 units of MMLV Reverse Transcriptase (1 μl; Gibco-BRL) in a solution containing 10 μl of RT-buffer, 14 μl of $H_2O$, 2.5 μl of spermidine (10mM), 0.5 μl of BSA (10 mg/ml), 10 μl of mixed dNTP (2 mM each; N = A, T, G and C), 10 μl of Triton X-100™ (10%, v/v), 1.5 μl of RNAse Block™ (Stratagene) and 1 μl of VH1FOR primer (50 pmol).

A portion (5 μl) of this solution containing Ig cDNA is subjected to in vitro amplification in 100 μl of solution containing: 62.5 μl $H_2O$, 10 μl of PCR-buffer, 10 μl of a mixture of dNTP (2 mM each, N = A, T, G and C), 10 ml of dimethyl sulphoxide (Merck) and 2 μl of primers VH1FOR and VH1BACK (50 pmol in $H_2O$). The solution is mixed and heated to 93°C for 3 min, cooled to 37°C, 0.4 μl of AmpliTaq™ DNA polymerase (Perkin Elmer Cetus) are added, and the solution is overlaid with 100 μl of paraffin oil in a 1 ml Eppendorf™ tube. The solution is then incubated in a temperature cycler (Intelligent Heating Block, Hybaid) as follows: 71°C for 0.2 min, followed by 93°C for 0.01 min, followed by 37°C for 0.2 min (4 cycles); 71°C for 0.2 min, followed by 93°C for 0.01 min, followed by 62°C for 0.2 min (30 cycles); 71°C for 3 min, followed by 62°C for 0.2 min, followed by 71°C for 3 min to complete final synthesis of DNA chains. One-tenth volume of the solution is analyzed by electrophoresis on 1% agarose gels containing ethidium bromide in order to visualize amplified DNA products. Successful selective amplification of Ig H-chain sequences results in a DNA band of ca. 350 bp, viewed under u.v. illumination. Occasionally other irrelevent side products of the amplification reaction are observed as DNA bands of different size.

Reverse transcription and in vitro amplification of Ig L-chain mRNA is performed as above for H-chain mRNA, except that for the reverse transcription step the VH1FOR primer is replaced by the M/Cκ oligonucleotide primer, and for the in vitro amplification step the VH1FOR and VH1BACK primers are replaced by VK1FOR and VK1BACK oligonucleotide primers. Successful amplification is monitored as described above for H-chain sequences, using agarose gel electrophoresis, and is indicated by the presence of a ca. 320 bp DNA band under u.v. illumination.

In both cases the amplified material is purified by extraction first with phenol/$CHCl_3$, and then $CHCl_3$. The material is finally precipitated with 2 volumes of ethanol at -20°C in the presence of 0.3 M NaOAc, pH 7.0, washed with 70% ethanol at the same temperature, and the DNA pellet air-dried.

1.3 Cloning of amplified Ig H- and L-chain cDNA sequences:

Amplified H- and L-chain cDNA (see example 1.2) is blunt-end cloned after preparing the ends of the DNA fragments by treatment with Klenow DNA polymerase and polynucleotide kinase.

DNA fragments prepared according to example 1.2 are dissolved in TE-buffer (20 μl), added separately to 17 μl of $H_2O$, 5 μl of mixed dNTP (1 mM each, N = A, T, G and C) and 5 μl of NT-buffer, and treated with 15 units (3 μl) of Klenow fragment DNA polymerase I (Boehringer) at room temperature for 30 min. The enzyme is inactivated by heating the solution at 65°C for 10 min, and the treated DNA samples are electrophoresed using TAE-buffer on 1% agarose gels containing ethidium bromide. DNA bands of ca. 350 bp for amplified H-chain DNA and ca. 320 bp for amplified L-chain DNA, visualised under u.v. illummination, are excised from the gel with a scalpel and purified on separate GENECLEAN™ columns (BIO 101 Inc.) according to the manufacturer's instructions. Each DNA is eluted in 30 μl of manufacturer's elution buffer, to which are added 7.5 μl of $H_2O$.

The purified H- and L-chain cDNA fragments (37.5 μl) are made up separately to 49 μl by the addition of

5 μl of PNK-buffer, 0.5 μl of dithiothreitol, 1 μl of spermidine (50 mM) and 5 μl of ATP (10 mM), and are each treated with 1 μl of T4 polynucleotide kinase (10 units; Pharmacia) at 37°C for 30 min. The reactions are terminated by the addition of 5 μl of 0.5 mM disodium-EDTA, pH 7.5, followed by 35 μl of TE-buffer and 10 μl of 3 M sodium acetate, pH 7.0, after which the DNA solutions are extracted with phenol/CHCl$_3$, CHCl$_3$, and precipitated with 2.5 volumes (v/v) of 95% ethanol. After centrifugation, supernatants are removed and DNA pellets dried and dissolved in 5 μl of TE-buffer.

DNA fragments are cloned in SmaI-digested, dephosphorylated BLUESCRIPT™ KS+ vector (Stratagene). Portions (2 μl) of the polynucleotide kinase-treated H- or L-chain cDNA fragments are separately ligated to 100 ng (1 μl) of prepared BLUESCRIPT™ vector in the presence of 1 μl of DNA ligase buffer, 1 μl of 10 x dithiothreitol (0.1 M), 0.5μl of 20 x ATP (10 mM), 3.5 μl of H$_2$O, and 1 μl (400 units) of T4 DNA ligase (New England Biolabs.) for 4 h at 14°C.

DNA ligation products are transformed into competent cells of E. coli K12/BZ234 prepared using standard procedures. Ampicillin-resistant clones are picked and plasmid DNA is prepared. Clones with appropriate-sized DNA inserts can be identified by co-digestion of plasmid DNAs with EcoRI + XbaI, which cut the vector polylinker sequence on opposite sides of the SmaI cloning site, and analysing DNA fragments by electrophoresis on 1 % agarose gels containing ethidium bromide.

## 1.4 Identification of functional Ig H- and L-chain gene rearrangements:

The nucleotide sequence of cloned plasmids with the correct sized DNA inserts are determined on double-stranded DNA templates with the SEQUENASE™ system using T3 and T7 oligonucleotide primers and the manufacturer's protocol (United States Biochemical). Several plasmid clones with identical sequences are obtained. Sequences of typical clones (F5-444LPCR39 and F5-444HPCR3) are listed as SEQ ID NO: 1 and Seq ID NO: 2 for L- and H-chain, respectively. Sequences shown include the oligonucleotide primers used for the selective in vitro amplification of Ig-related sequences, the position of DNA restriction sites located within the primers, and the location of complementarity-determining regions (CDRs) of the Ig L- and H-chain V-regions, deduced from comparison with the data base of murine Ig V-region sequences (Kabat et al., "Sequences of proteins of immunological interest", fourth edition, U.S. Dept. Health & Human Services, 1987).

The V-region rearrangement of F5-444LPCR39 uses the J5 L-chain J-minigene joining exon (beginning at nucleotide position 283; SEQ ID NO: 1 ) and includes a continuous open reading frame encoding a polypeptide sequence formed by V-J exon fusion, characteristic of a functional Ig L-chain gene rearrangement.

The V-region rearrangement of F5-444HPCR3 uses the J$_H$2 H-chain J-minigene joining exon (beginning at nucleotide position 308; SEQ ID NO: 2) and includes a continuous open reading frame encoding a polypeptide sequence formed by V-D-J exon fusion, characteristic of a functional Ig H-chain gene rearrangement.

Occasional other DNA clones are obtained using F5-444 cell mRNA, none of which resemble Ig H- or L-chain V-region sequences. The sequences of at least three independent cloned plasmids containing H- and L-chain sequences are compared and shown to be identical, to reduce the possibility that sequences resulting from in vitro amplification contain point mutations in the V-region sequences.

## Example 2. Construction of cloning vectors for DNA manipulation

### 2.1 Vector KS+exPvuII:

In order to facilitate cloning F5-444LPCR39 sequences for expression in BLUESCRIPT™ KS+ plasmid vector (Stratagene), the (two) non-essential PvuII restriction sites are eliminated. BLUESCRIPT™ KS+ plasmid DNA (10 μg) is digested to completion with PvuII to generate two linear DNA fragments of ca. 2300 and 360 bp. The DNA fragments are separated by electrophoresis on 1% agarose gels containing ethidium bromide, and purified using GENECLEAN™ (BIO 101 Inc.). This provides ca. 150 ng of purified 360 bp fragment and ca. 600 ng of 2300 bp fragment. The larger (2300 bp) fragment is treated with 20 units of calf intestinal alkaline phosphatase (CIAP; Boehringer). The CIAP-treated fragments are then extracted with phenol/CHCl$_3$, then CHCl$_3$, precipitated with 0.54 volumes of isopropanol in the presence of 0.3 M NaOAc, pH 7.0, and the DNA pellet is washed with 70% (v/v) ethanol at -20°C. The pellet is air-dried and dissolved in 6 μl of TE-buffer.

Oligonucleotide linkers (10 ng in 1 μl of H$_2$O) containing an NcoI DNA restriction site (pCCCATGGG; New England Biolabs.) are treated for 30 min at 37°C with 1 μl of T4 polynucleotide kinase (PL Biochemicals) in a solution containing 36.5 μl of H$_2$O, 5 μl of 10 x PNK-buffer, 0.5 μl of 0.1 M dithiothreitol, 1 μl of 50 mM spermidine and 5 μl of 10 mM ATP. The phosphorylated linkers are added to 1 μl (150 ng) of the 360 bp PvuII-digested KS+ plasmid DNA fragment and 5 μl of CIAP-treated 2300 bp fragment (200 ng). The mixture of fragments is extracted with pheno/CHCl$_3$, then CHCl$_3$, and precipitated with ethanol in the presence of 0.3 M NaOAc, pH 7.0 at -20°C, washed with 70% (v/v) ethanol, air-dried and dissolved in 0.5 ml of $H_2O$. The plasmid DNA/linker mixture is ligated overnight at 4°C using 10,000 units/5 µl of T4 DNA ligase (New England Biolabs.) in a total volume of 1 ml of solution containing 100 µl of DNA ligase buffer, 100 µl of 0.1 M dithiothreitol, 50 µl of 20 mM ATP and 245 µl of $H_2O$. Ligation products are transformed into E. coli K12/BZ234, ampicillin-resistant colonies selected and plasmid DNA preparations made using standard procedures (Sambrook et al., op. cit., Section 1.82-84 & Section 1.25-28). Plasmids having acquired NcoI-linkers ligated at both of the junctions between the 360 bp and 2300 bp DNA fragments are detected by DNA restriction analysis using NcoI.

The NcoI restriction sites are removed from one such plasmid by digesting to termination with NcoI, treating a sample of the digested DNA with Klenow DNA polymerase to generate blunt-end fragments and eliminate the NcoI "sticky-ends", and religation of the fragments using T4 DNA ligase, all using standard procedures as described above and in example 1.3.

The final plasmid, designated KS+exPvuII, contains NcoI linkers at the original locations of the PvuII restriction sites in the KS+ vector, in which the NcoI restriction sites have been eliminated using Klenow DNA polymerase. The relative orientation of the 360 bp and 2300 bp KS+ DNA fragments and the polylinker DNA cloning sites remain the same as in the BLUESCRIPT™ KS+ vector.

## 2.2 Vectors KS+VHX-Vec and KS+VKX-Vec:

The vectors M13-VHPCR1 and M13-VKPCR1 (Orlandi et al., Proc. Natl. Acad. Sci. USA 86, 3833-3837, 1989, made available by The Medical Research Council, 20 Park Crescent, London W1N 4AL) contain 817 bp and 648 bp BamH1/HindIII restriction fragments, respectively, capable of accepting Ig H- and L-chain V-region DNA sequences amplified from F5-444 cell RNA using oligonucleotides as described in examples 1.2 and 1.3. These BamHI/HindIII vector DNA fragments contain mouse Ig promoters, leader peptide exons and functional rearranged V-D-J and V-J V-region exons.

This DNA is transformed into E. coli K12/TG1, and plasmid DNAs are prepared using standard procedures: Each bacteriophage DNA preparation is digested separately to termination using BamHI + HindIII, and the smaller Bam/HindIII fragment isolated in each case (817 bp for M13-VHPCR1, and 648 bp for M13-VKPCR1) by electrophoresis on 0.8% agarose gels containing ethidium bromide. DNA bands of the correct size, visualized under u.v. illumination, are excised from the gel and DNA recovered by electroelution from the agarose followed by phenol/$CHCl_3$ extraction and precipitation with isopropanol. DNA pellets are dried and dissolved separately in 10 µl of TE-buffer.

## 2.2.1 Vector KS+VHX-Vec:

In M13-VHPCR1 the BamHI/HindIII segment contains unique internal DNA restriction sites for enzymes PstI and BstEII, allowing removal of the "irrelevant" Ig H-chain V-region and replacement by a V-region sequence such as that located between PstI and BstEII restriction sites of F5-444HPCR3. The exact location of PstI and BstEII sites in the DNA amplification primers VH1FOR and VH1BACK ensure that the H-chain polypeptide-coding sequence is maintained in the correct translational frame with the surrounding DNA sequences, facilitating expression of the V-region protein.

The 817 bp M13-VHPCR1 DNA fragment is cloned after ligation to BLUESCRIPT™ KS+ vector. The vector is prepared for use by digestion with restriction enzymes BamHI + HindIII, followed by extraction with phenol/$CHCl_3$ and $CHCl_3$, ethanol precipitation, centrifugation, washing with 70% ethanol, drying the DNA pellet and dissolving in TE-buffer. Ligation is carried out for 6 h at 14°C in 15 µl of solution containing 2 µl of BamHI + HindIII-treated vector DNA (40 µg), 2,5 µl of M13-VHPCR1 DNA fragment (2,5 µl), 1,5 µl of 10 mM ATP, 1,5 µl of 0,1 M dithiothreitol, 1,5 µl of DNA ligase buffer, 4,5 µl of TE-buffer and 1.5 µl (600 units) of T4 DNA ligase (New England Biolabs.). Ligation products are transformed into E. coli K12/TG1, ampicillin-resistant colonies selected and plasmid DNAs prepared using standard procedures (Sambrook et al., op. cit., Section 1.82-84 & 1.25-28). A clone is selected containing a recombinant plasmid with a BamHI/HindIII insert DNA fragment of 817 bp, and its sequence confirmed as corresponding to the expected fragment derived from M13-VHPCR1 using the SEQUENASE™ system with T3/T7 oligonucleotide primers, using conditions provided by the manufacturer (United States Biochemical). This plasmid is referred to as KS+VH-Vec.

KS+VH-Vec is further modified by the introduction of a second XbaI DNA restriction site in addition to the one originally derived from the BLUESCRIPT™ KS+ polylinker. KS+VH-Vec DNA is digested to termination with restriction endonuclease HindII, and the fragments are dephosphorylated with calf intestinal alkaline phosphatase using standard procedures (see example 2.1). After treatment the DNA is dissolved in TE-buffer at a concentration of 120 ng/ml. After dissolution, the DNA fragments (0.5 µl) are added to 1 µl of XbaI oligonucleotide linkers (pCTCTAGAG, 500 ng/µl; New England Biolabs.), 1.5 µl of 10 mM ATP, 1.5 µl of 0.1 M dithiothreitol, 1.5 µl of DNA ligase buffer and 7.5 µl of TE-buffer, and ligated overnight at 14°C using 1.5 µl (600 units) of T4 DNA ligase (New England Biolabs.). Following ligation, the mixture is used to transform E. coli K12/TG1, ampicillin-resistant colonies are selected and plasmid DNA preparations made using standard procedures, as described above (Sambrook et al., op. cit., Section 1.82-84 & 1.25-28). Plasmids having acquired the XbaI-linker sequence are detected by digestion using restriction endonuclease XbaI. One plasmid is selected and its nucleotide sequence confirmed as having an additional XbaI-linker sequence incorporated at the original HindII restriction site in the BLUESCRIPT™ KS+ sequence. The plasmid is referred to as SK+VHX-Vec.

2.2.2 Vector KS+VKX-Vec:

In M13-VKPCR1, the BamHI/HindIII segment contains unique internal DNA restriction sites for enzymes PvuII and BclI, allowing removal of the "irrelevant" Ig L-chain V-region and replacement by a V-region sequence such as that located between PvuII and BglII restriction sites of F5-444LPCR3. The exact location of PvuII and BglII sites in the DNA amplification primes VK1FOR and VK1BACK ensures that the L-chain polypeptide-coding sequence is maintained in the correct translational frame with the surrounding DNA sequences, facilitating expression of the V-region protein. Since the DNA restriction enzyme BclI is sensitive to Dam+methylation, an appropriate Dam⁻ host strain, e.g. E. coli K12/BZ103, K12/E3225 or K12/R832, must be used for growth of phage and plasmids where it is intended, as here, to utilize the BclI restriction site for cloning purposes.

The 648 bp BamHI/HindIII DNA fragment of M13-VKPCR1 is cloned in a similar manner to that of M13-VHPCR1 (example 2.2.1) by ligation to BamHI/HindIII-digested KS+exPvuII vector (described in example 2.1). After transformation colonies are selected, and one containing a recombinant plasmid with a BamHI/HindIII insert fragment of 648 bp is analyzed and its sequence confirmed as corresponding to the expected fragment derived from M13-VKPCR1 using the SEQUENASE™ system with T3/T7 oligonucleotide primers as described above. This plasmid is referred to as KS+VK-Vec.

The KS+VK-Vec vector is further modified by introduction of an XbaI-linker at the HindII restriction site in the BLUESCRIPT™ KS+ polylinker sequence, and its sequence confirmed in a similar manner to that described above for KS+VHX-Vec (example 2.2.1). Plasmid DNA is transformed into E. coli K12/R832 (Dam⁻) in order to maintain its BclI restriction site in the unmethylated state for cloning purposes. The final plasmid vector is referred to as KS+VKX-Vec.

Example 3. Construction of a vector for Ig H-chain gene expression in myeloma cells

3.1 Isolation of a DNA fragment encoding a human γ1 H-chain:

A human DNA library is constructed in the bacteriophage λ vector Charon 4a by limited digestion of human fetal liver DNA with restriction endonucleases HaeIII and AluI using published procedures (Lawn et al., Cell 15, 1157, 1978). Approximately 1 x 10⁶ independent recombinant phages are plated on E. coli K12/803 and screened for the presence of human Ig H-chain DNA sequences using an homologous murine Ig H-chain DNA probe.

A nick-translated ³²P-labelled mouse IgG H-chain DNA probe corresponding to the XbaI/HhaI fragment of the mouse Ig γ2b gene locus is used to screen recombinant phage as described previously (Takahashi et al., Cell 29, 671 -679, 1982). One DNA clone analyzed (#95/4) contains a 7 kb HindIII DNA segment encompassing the human IgG1 constant region exons CH1, hinge region, CH2 and CH3, as determined by restriction mapping and by nucleotide sequence analysis. The portion of clone #95/4 that is sequenced corresponds to the published human IgG1 gene sequence (EMBL data base sequence entry HUMIGCC4) which starts from one of the terminal (HindIII) restriction sites of the 7 kb HindIII segment (Ellison et al., Nucleic Acids Res. 10, 4071-4079, 1982).

The 7 kb HindIII DNA fragment from clone #95/4 containing the human IgG1 constant region exons is subcloned into the HindII site of dephosphorylated BLUESCRIPT™ KS+ vector and transformed into E. coli K12/TG1. Ampicillin-resistant colonies are picked, plasmid DNAs prepared and those containing the subcloned 7 kb DNA fragment isolated using standard procedures (Sambrook et al., op. cit., Section 1.82-84 & 1.25-28). Plasmid DNA from one clone is identified in which the HindIII site located 5′ of the human IgG1 CH1 exon is oriented next to the XbaI DNA restriction site of the polylinker of BLUESCRIPT™ KS+. The plasmid is designated γ1-KS+.

The entire 7 kb DNA insert of γ1-KS+ containing the human IgG1 coding sequences is recovered by electrophoresis on 1% agarose gels containing ethidium bromide after digestion of γ1-KS+ using EcoRI/XbaI which cleaves the KS+ polylinker on both sides of the human DNA insert (neither enzyme cleaves the 7 kb HindIII insert DNA fragment of γ1-KS+). The DNA fragment is electroeluted, extracted with phenol/CHCl₃, then

$CHCl_3$, and recovered by ethanol precipitation/centrifugation using standard procedures. This DNA is referred to as Fragment 1. The DNA is dissolved in TE-buffer and stored at -20°C.

3.2 Cloning of a DNA fragment containing the murine Ig H-chain transcriptional enhancer:

A 686 bp XbaI/EcoRI DNA fragment corresponding to nucleotides 2877-3563 of the murine Ig H-chain gene locus (GENBANK data base entry MUSSIGCD07) containing the murine Ig H-chain transcriptional enhancer is cloned in EcoRI/XbaI-digested BLUESCRIPT™ KS+ vector and its sequence verified using the SEQUENASE™ system with T3 and T7 olignonucleotide primers. The plasmid is designated pDA24/3. The insert DNA fragment of pDA24/3 is isolated after digestion with EcoRI/bal, and recovered after electrophoresis on 1% agarose gels containing ethidium bromide using the same procedure used for Fragment 1. The DNA is dissolved in TE-buffer and stored at -20°C. This DNA is referred to as Fragment 2.

3.3 Assembly of plasmid vector Huγ1-EH-gpt:

DNA Fragment 1 and Fragment 2 (examples 3.1 and 3.2) are cloned by coligation into vector pSV2gpt (Southern & Berg, J. Mol. App. Genet. 1, 327, 1982), linearised by digestion using EcoR1. The DNA ligation mixture contains digested pSV2gpt vector DNA (50 ng in 1 μl of TE-buffer), Fragment 1 (70 ng in 1.3 μl of TE-buffer), Fragment 2 (7 ng in 1.0 μl of TE-buffer), 1.5 μl of 10 mM ATP, 1.5 μl of 0.1 M dithiothreitol, 1.5 μl of DNA ligase buffer, 5.7 μl of TE-buffer and 1.5 μl of T4 DNA ligase (600 units; New England Biolabs.). Ligation is performed overnight at 14°C. After transformation into E. coli K12/TG1, ampicillin-resistant recombinants are identified and plasmid DNAs prepared using standard procedures (Sambrook et al., op. cit., Section 1.82-84 & 1.25-28). DNA clones are selected based on the results of restriction analysis using the enzymes EcoRI, BamHI, HindIII, PvuII and XbaI. A plasmid with the appropriate DNA restriction properties is selected and designated Huγ1-EH-gpt. The plasmid contains pSV2gpt sequences and the mouse Ig H-chain enhancer and human IgG1 constant region exons separated by a unique XbaI cleavage site.

Example 4. Construction of a vector for Ig L-chain gene expression in myeloma cells

4.1 Isolation of a DNA fragment encoding the human Cκ L-chain constant region exon:

A nick-translated $^{32}$P-labelled murine Ig germline L-chain J-region genomic DNA probe is prepared, corresponding to a ca. 2240 bp HindIII/XbaI segment of Balb/c mouse liver DNA (nucleotide positions 659-2900 of the published germline Ig L-chain locus; Max et al., Proc. Natl. Acad. Sci. USA 76, 3450-3454, 1979; EMBL data base sequence entry MUSIGKJC2). The probe is used to screen a human recombinant λ phage DNA library for segments containing homologous human Cκ L-chain DNA sequences, using a similar procedure to that described for Ig H-chain sequences (example 3.1). A positively-hybridizing recombinant phage is selected and DNA prepared using standard procedures.

A DNA fragment is subcloned from the cross-hybridizing recombinant phage, corresponding to a ca. 2.5 kb BalI/EcoRI fragment containing the human Ig Cκ constant region exon (Hieter et al., Cell 22, 197-207, 1980). The BalI DNA restriction site and Cκ coding region are indicated in the EMBL data base sequence entry HUMIGKC3; BalI DNA cleavage site = nucleotide position 31; Cκ coding region = nucleotide position 334-656. The cloning vector used is SmaI/EcoRI-digested BLUESCRIPT™ KS+, and the methods used are standard cloning procedures (Sambrook et al., op. cit., Section 1.63-70). A recombinant plasmid with the desired DNA restriction characteristics is selected, and its structure confirmed by partially sequencing using the SEQUENASE™ system with T3 and T7 oligonucleotide primers according to the manufacturer's protocol. The plasmid is designated pDA27.

pDA27 contains two DNA restriction sites for the enzyme XbaI; one derived from the KS+ polylinker, the second being a site in the human Cκ DNA insert of pDA27 located ca. 1 kb from the EcoRI site. In order to facilitate subsequent use in the construction of expression vectors the latter DNA restriction site is eliminated as follows: pDA27 DNA (30 μg) is partially digested with 4 μl of XbaI (1 unit/μl; Boehringer) for 45 min at 37°C. The DNA is then extracted with phenol/$CHCl_3$, $CHCl_3$, ethanol precipitated and dissolved in 82 μl of distilled $H_2O$ to which is added 10 μl of NT-buffer, 4 μl of mixed dNTPs (2 mM each; N = A, T, G and C). Klenow fragment DNA polymerase (4 μl; 4.9 units/μl; Boehringer) is added and the mixture incubated at room temperature for 30 min, after which the enzyme is heat-inactivated by heating at 65°C for 5 min. Partial digestion generates DNA fragments of ca. 5.5 kb which are separated from terminal digestion products by electrophoresis on a 0.8% agarose gel containing ethidium bromide. After electrophoresis the 5.5 kb DNA band is electroeluted from the agarose gel and recovered by phenol / chloroform extraction and ethanol precipitation as described above. The DNA pellet is dissolved in 20 μl of TE-buffer (yield approx. 40 ng). One-half of the material is religated, transformed into E. coli K12/BZ234, and ampicillin-resistant colonies are selected. Plasmid DNA is prepared from appropriate clones and digested with a combination of EcoRI + XbaI. Plasmids with the correct XbaI site eliminated generate two EcoRI/XbaI DNA restriction fragments; one of ca. 3 kb (containing KS+ vector sequences) and one of 2.5 kb (human Cκ-containing fragment). One such plasmid is selected and referred to as pDA28.

The 2.5 kb human DNA fragment of pDA28, now containing an XbaI DNA restriction site (derived from the KS+ polylinker) located 5′ of the human Cκ-coding region, is recovered from the plasmid by digestion with EcoRI/XbaI, and purified by electrophoresis on 0.8% agarose gels followed by extraction with phenol/CHCl$_3$, then CHCl$_3$, ethanol precipitation, drying and dissolution in TE-buffer. The DNA is referred to as Fragment 3.

## 4.2 Assembly of plasmid vector HuCκ-EH-neo:

DNA Fragment 2 (ca. 700 bp EcoRI/XbaI DNA fragment containing the murine Ig H-chain transcriptional enhancer, example 3.2) and Fragment 3 (ca. 2.5 kb EcoRI/XbaI DNA fragment containing the human Cκ coding region; example 4.1) are cloned by coligation into vector pSV2neo (Southern & Berg, J. Mol. App. Genet. 1, 327, 1982), linearized by digestion using EcoRI. DNA is ligated overnight at 14°C in a mixture containing 20 ng of EcoRI-digested pSV2neo DNA (1 μl), 15 ng of DNA Fragment 2 (1 μl), 25 ng of DNA Fragment 3 (0.5 μl), 2 μl of DNA ligase buffer, 2 μl of 10 mM ATP, 2 μl of 0.1 M dithiothreitol and 10 μl of H$_2$O, using 1 μl of T4 DNA ligase (400 units/μl; New England Biolabs.). The ligation mixture is transformed into E. coli K12/803, ampicillin-resistant colonies are selected, and plasmid DNAs prepared using standard procedures (Sambrook et al., op. cit., Section 1.82-84 & 1.25-28). Plasmids are analyzed by digestion with EcoRI/XbaI and Pst 1, and one with the desired orientation of DNA fragments selected, referred to as HuCκ-EH-neo.

## Example 5. Vectors for expression of chimeric mouse/human antiidiotypic monoclonal antibody in myeloma cells

### 5.1 Generation of the chimeric Ig H-chain expression vector F5-444-Huγ1:

Vector KS+VHX-Vec (7.5 μg; example 2.2.1) is digested to termination using restriction endonucleases BstEII/XbaI, and the DNA fragments fractionated on a 0.8% agarose gel containing ethidium bromide. Digestion releases the "irrelevant" V-region gene segment (ca. 330 bp) and a ca. 3.3 kb BstEII/XbaI vector DNA fragment, which is excised from the gel, recovered by electroelution, extracted with phenol/CHCl$_3$, then CHCl$_3$, and precipitated with ethanol. The DNA pellet obtained after centrifugation is washed in 70% ethanol at -20°C and dissolved in distilled H$_2$O.

Plasmid F5-444HPCR3 (ca. 30 μg; example 1.4) is digested to completion with restriction endonucleases BstEII/PstI, releasing a ca. 330 bp DNA fragment containing the cloned F5-444 Ig H-chain V-region (SEQ ID NO:2). The fragment is separated from vector sequences by electrophoresis on a 1% agarose gel, recovered by fractionation using GENECLEAN™ (BIO 101 Inc.) using the procedure provided by the manufacturer. DNA is precipitated at -20°C with 2.5 volumes of 95% ethanol in the presence of 0.3 M NaOAc, pH 7.0, washed using 70% ethanol at the same temperature, and dissolved in TE-buffer. The yield is ca. 600 ng of DNA.

The isolated ca. 3.3 kb vector DNA fragment (100 ng in 2 μl of H$_2$O) isolated from KS+VHX-Vec as described above is ligated overnight at 4°C together with the isolated ca. 330 bp F5-444 Ig H-chain V-region DNA fragment (10 μg in 1 μl of TE-buffer) in the presence of 1 μl of DNA ligase buffer, 1 μl of 0.1 M dithiothreitol, 0.5 μl of 20 mM ATP, 3.5 μl of H$_2$O and 1 μl of T4 DNA ligase (400 units/μl; New England Biolabs.). DNA ligation products are transformed into E. coli K12/BZ234, ampicillin-resistant colonies selected, and plasmid DNA clones identified. Several clones are identified with the expected DNA restriction properties, and one clone containing the F5-444 Ig H-chain V-region sequenced using the SEQUENASE™ system with T3/T7 oligonucleotide primer, using the manufacturer's protocol. The DNA clone is referred to as clone #782.

Clone #782 contains the F5-444 Ig H-chain V-region adapted for expression by incorporation into the M13-VHPCR1 cassette (described in example 2.2). The adapted H-chain V-region exon, plus the H-chain leader peptide exon and promoter element derived from the M13-VHPCR1 cassette, are released together from clone #782 on a ca. 830 bp DNA fragment by digestion with XbaI. The XbaI fragment is separated from the larger vector-containing DNA fragment by electrophoresis on a 0.8% agarose gel, purified by phenol/CHCl$_3$ extraction and ethanol precipitation by standard procedures, and dissolved in TE-buffer. The DNA fragment is ligated to plasmid vector Huγ1-EH-gpt (example 3.3) as follows: 200 ng of XbaI-digested, dephosphorylated Huγ1-EH-gpt (in 2 μl of TE-buffer) and 10 ng of isolated DNA fragment (in 0.5 μl of TE-buffer) are added to 2 μl of DNA ligase buffer, 2 μl of 0.1 M dithiothreitol, 1 μl of 20 mM ATP and 10.5 μl of H$_2$O. DNA ligation is performed overnight at 4°C using 2 μl of T4 DNA ligase (800 units; New England Biolabs.). DNA ligation products are transformed into E. coli K12/BZ234, ampicillin-resistant colonies selected and plasmid DNAs prepared using standard procedures (Sambrook et al., op. cit., Section 1.82-84 & 1.25-28). Plasmids containing the inserted ca. 830 bp XbaI DNA fragment in the required orientation for expression are detected by using BstEII. Plasmid DNA is prepared from one DNA clone, referred to as F5-444-Huγ1H808.

## 5.2 Generation of the chimeric Ig L-chain expression vector F5-444-HuCκ:

Vector KS+VKX-Vec (60 μg; example 2.2.2) is digested to termination using restriction endonucleases BclI/PvuII, dephosphorylated by treatment with calf intestinal alkaline phosphatase using a standard procedure (see example 2.1) and the DNA fragments fractionated on a 0.8% agarose gel containing ethidium bromide using TBE-buffer. Digestion releases the "irrelevant" V-region gene segment (ca. 300 bp) and a ca. 3.3 kb BclI/PvuII vector DNA fragment, which is excised from the gel, recovered by electroelution, extracted with phenol/CHCl$_3$, then CHCl$_3$, and precipitated with isopropanol. The DNA pellet obtained by centrifugation is washed in 70% ethanol at -20°C and dissolved in 100 μl of TE-buffer.

Plasmid F5-444LPCR39 (example 1.4) is digested to completion with restriction endonucleases BglII/PvuII, releasing a ca. 320 bp DNA fragment containing the cloned F5-444 Ig L-chain V-region (SEQ ID NO:1). The fragment is separated from vector sequences by electrophoresis on a 0.8% agarose gel, recovered by electroelution, extracted with phenol/CHCl$_3$, then CHCl$_3$, precipitated at -20°C with 2.5 volumes of 95% ethanol in the presence of 0.3 M NaOAc, pH 7.0, washed using 70% ethanol at the same temperature, and dissolved in TE-buffer.

The ca. 3.3 kb dephosphorylated vector DNA fragment (200 ng in 2 μl TE-buffer), isolated from KS+VKX-Vec as described above, is ligated together with the isolated ca. 320 bp F5-444 Ig L-chain V-region DNA fragment (50 ng in 1 μl of TE-buffer) in the presence of 2 μl of 0.1 M dithiothreitol, 1 μl of 20 mM ATP, 10 μl of H$_2$O, 2 μl of DNA ligase buffer and 2 μl (800 units; New England Biolabs.) of T4 DNA ligase. DNA ligation products are transformed into E. coli K12/BZ234, ampicillin-resistant colonies selected, plasmid DNA isolated and clones identified by restriction analysis using XbaI, and sequence analysis performed using the SEQUENASE™ system with T3 and T7 oligonucleotide primers according to the manufacturer's protocol. One clone is selected. The DNA clone is referred to as clone #820.

Clone #820 contains the F5-444 Ig L-chain V-region adapted for expression by incorporation into the M13-VKPCR1 cassette (described in example 2.2). The adapted L-chain V-region exon, plus the L-chain leader peptide exon and promoter element derived from the M13-VKPCR1 cassette, are released together from clone #820 on a ca. 630 bp DNA fragment by digestion with XbaI. This smaller XbaI DNA fragment is separated from the larger vector-containing DNA fragment by electrophoresis on a 0.8% agarose gel, purified by phenol/CHCl$_3$ extraction and ethanol precipitation by standard procedures, and dissolved in TE-buffer. The DNA fragment is ligated to plasmid vector HuCκ-EH-neo (example 4.2) as follows: 100 ng of XbaI-digested, dephosphorylated HuCκ-EH-neo (in 1 μ of TE-buffer) and 20 ng of isolated DNA fragment (in 4 μl of TE-buffer) are added to 1 μl of DNA ligase buffer, 1 μl of 0.1 M dithiothreitol, 0.5 μl of 20 mM ATP, 1.5 μl of H20, and 1 μl of T4 DNA ligase (400 units; New England Biolabs.). Ligation is performed for 2 h at 15°C. DNA ligation products are transformed into E. coli K12/BZ234, ampicillin-resistant colonies selected and plasmid DNAs prepared using standard procedures (Sambrook et al., op. cit., Section 1.82-84 & 1.25-28). Plasmids containing the inserted ca. 630 bp XbaI DNA fragment in the required orientation for expression are detected by digestion using restriction endonuclease PstI. DNA from one such plasmid is prepared, and is referred to as F5-444-HuCκ828.

## Example 6. Transfection of Sp2/O myeloma cells using F5-444-Huγ1H808 and F5-444-HuCκ828

### 6.1 Growth and preparation of cells:

Sp2/0 (ATCC CRL 1581) is a well-characterized mouse cell-line of lymphoid origin. It is an Ig non-secreting variant of a cell-line obtained from the fusion of a mouse spleen cell with the myeloma X63-Ag8, a subline of the myeloma MOPC-21 (Köhler and Milstein, Eur. J. Immunol. 6, 511, 1976; Shulman et al., Nature 276, 270, 1978). Sp2/0 cells are grown at 37°C in HB101™ medium (Hana Biologics), supplemented with 5% (v/v) FCS (Amimed), 1 mM sodium pyruvate (Gibco), 2 mM L-glutamine (Amimed), 10 mM HEPES buffer, pH 7.4 (Serva) and 10 μg/ml gentamycin (Sigma).

Cells are grown in a humidified atmosphere of air and 5% CO$_2$ in a tissue culture incubator (Brouwer, model 3035) in 175 cm$^3$ tissue culture flasks (Costar, 3151). Cells are harvested by gentle centrifugation in 50 ml sterile tubes (Falcon 2070) at 4°C (200 x g, 800 rev/min; Beckman J-6B/P centrifuge), frozen and stored at -120°C in HB101™ (Hana Biologics) containing 15% FCS (Amimed) and 7.5% (v/v) dimethyl sulphoxide (Serva) in clean, sterile plastic capped tubes.

24 h before transfection, Sp2/0 cells are passaged into fresh growth medium at a concentration of $1 \times 10^5$ cells/ml. Immediately before transfection, cells are harvested as described above, and the cell pellets (total no. of cells ca. $9 \times 10^7$) washed twice in PBS-CM at 4°C and resuspended in the same buffer at a concentration of ca. $1 \times 10^8$ cells/ml.

## 6.2 Preparation of DNA fragments and transfection:

For transfection, plasmid DNAs F5-444-Hu$\gamma$H808 (example 5.1) and F5-444-HuC$\kappa$828 (example 5.2) are digested to termination using restriction endonuclease EcoRI to release the chimeric mouse:human Ig H- and L-chain insert DNA fragments. Digested DNA is extracted with phenol/CHCl$_3$, then CHCl$_3$, precipitated at -20°C with ethanol in the presence of 0.3 M NaOAc, pH 7.0, washed with 70% ethanol at the same temperature, and resuspended in TE-buffer.

Samples (5 $\mu$g) of each digested plasmid DNA are combined (total volume of 15 $\mu$l in TE-buffer) and added to $2 \times 10^7$ (200 $\mu$l) of Sp2/0 cells at 0°C, previously grown, washed and resuspended in PBS-CM as described above (example 6.1). The cells plus DNA are drawn into the barrel of a TA750 electrotransfection apparatus (Kruess GmbH), pre-cooled to 0°C using ice-cold sterile PBS-CM. Cells are subjected to two electrical pulses of 3500 V/cm for 10 $\mu$s, with a 30 s interval between pulses, using the cylindrical electroporation chamber provided by the manufacturer. Cells are expelled gently into a clean, sterile cryotube (Nunc) and kept on ice for 10 min, after which they are diluted (1:3, v/v) into supplemented HB101™ growth medium (see above, example 6) containing 15% (v/v) FCS (Amimed) and incubated at room temperature for 20 min. The cells are then further diluted to 100 ml in the same growth medium and 1 ml samples distributed into wells of 24-well Nunclon™ tissue culture clusters (Delta). After incubation for 24 h at 37°C, as described above, 0.5 ml of the growth medium in each well are removed and replaced with 0.5 ml of prewarmed selection medium containing supplemented HB101™(Hana Biologics) + 15% (v/v) FCS (Amimed) supplemented with a 1:45 dilution of HT (Sigma, 50 x HT media supplement) and 1 mg/ml geneticin (Gibco). One-half (0.5 ml) of the medium is replaced every 48 h with additional selection medium, as described above, and selection continued for at least 14 days.

## Example 7. Identification of transfectomas secreting chimeric monoclonal antibody

Clones secreting Ig are identified by ELISA using anti-human Ig $\kappa$-chain and anti-human IgG antibodies as follows: 96-well microtitre plates (Nunclon™ maxisorp) are coated overnight with 50 $\mu$l of a 1:500 (v/v) dilution of goat anti-human $\kappa$ antibody (Tago, 4106) in PBS by incubation at 4°C. Plates are then washed six times with PBS + 0.05% Tween-20™ (Fluka), followed by incubation for 20 min at 37°C with RIA-buffer (10 $\mu$l/well). Plates are rewashed six times with PBS + 0.05% Tween-20™ (Fluka).

Cell-culture supernatants from transfectoma clones (example 6.1) are diluted using RIA-buffer, 50 $\mu$l samples added to each well, and the microtitre plates incubated for 2 h at 37°C. Following incubation with cell culture supernatants, plates are washed six times using PBS + 0.05% Tween-20™ (Fluka), after which 50 $\mu$l/well of alkaline phosphatase-conjugated goat anti-human IgG antibody in RIA-buffer (1: 1000, v/v dilution; Tago 2490) are added and the plates incubated 2 h at 37°C. Plates are again washed six times with PBS + 0.05% Tween-20™ (Fluka), and p-nitrophenyl phosphate substrate is added (100 $\mu$l/well; Sigma 104 phosphatase substrate tablets; 4 x 5 mg tablets dissolved in 20 ml of substrate buffer), after which the plates are incubated in the dark for 10 min at room temperature. The plates are blocked by incubation with 0.5 M NaOH (100 $\mu$l/well) and $A_{405}$ measured using a photometer (TITERTEK™, multiscan MCC). Concentrations of recombinant MAb are determined with reference to a standard consisting of dilutions of a purified mouse:human chimeric antibody of identical human isotype (IgG1,$\kappa$).

Cells from positive wells are cloned by limited dilution. Typical transfectomas produce under these conditions ca. 0.02-10 $\mu$g/ml of recombinant MAb/$1 \times 10^6$ cells after 6 days growth. One cell-line is selected (F5-CH$\gamma$1) which produces ca. 10 $\mu$g/ml recombinant MAb under these conditions.

## Example 8. Specificity of the mouse/human chimeric MAb F5-CH$\gamma$1

The murine anti-idiotypic MAb F5-444 recognizes idiotypic determinants on the murine MAb AC1.59 (see example 1). MAb AC1.59, a monoclonal antibody of IgM, $\kappa$ isotype, can thus be used as a means to compare the specificity of recombinant chimeric MAb F5-CH$\gamma$1 with the parental murine MAb F5-444.

Microtitre plates (96-well Nunclon™ maxisorp) are coated overnight with 50 $\mu$l of a 1:1000 (v/v) dilution of purified murine MAb AC1.59 (T. Crepaldi et al., Tissue Antigens 26, 25-34, 1985; stock concentration 3 mg/ml) in PBS, by incubation at 4°C. Plates are then washed six times with PBS + 0.05% Tween-20™ (Fluka), followed by incubation for 20 min at 37°C with RIA-buffer (100 $\mu$l/well). Plates are rewashed six times with PBS + 0.05% Tween-20™ (Fluka). Cell cultwe supernatants from (a) murine hybridoma F5-444, (b) the transfectoma F5-CHγ1, and (c) pwified F5-444 MAb (1.4 mg/ml) as standard and (d) isotype-matched chimeric MAb 4-8-13 (IgG1, κ) specific for carcinoembryonic antigen (1 mg/ml, N. Hardman et al., Int. J. Cancer 44, 424, 1989) as negative control are diluted separately using RIA-buffer, 50 μl added to microtitre wells, and the plates incubated for 2 h at 37°C. Following incubation, plates are washed six times using PBS + 0.05% Tween-20™ (Fluka), after which 50 μl/well of conjugated antibodies are added. Antibody conjugates are either alkaline-phosphatase conjugated goat anti-human IgG (H+L) (1:1000, v/v dilution; Jackson ImmunoResearch Labs.), or alkaline-phosphatase conjugated rat anti-mouse IgG (H+L) (1:1000, v/v dilution; Jackson ImmunoResearch Labs.). Plates are then incubated for 2 h at 37°C. Plates are again washed six times with PBS + 0.05% Tween-20™ (Fluka), and p-nitrophenyl phosphate substrate is added (100 μl/well; Sigma 104 phosphatase substrate tablets; 4 x 5 mg tablets dissolved in 20 ml of substrate buffer), after which plates are incubated in the dark for 10 min at room temperature. The plates are blocked by incubation with 0.5 M NaOH (100 μl/well), and $A_{405}$ is measured using a photometer (TITERTEK™, multiscan MCC). Results are shown in the following table:

| Developing Antibody | (a) F5-444 supernatant | (b) F5-CHγ1 supernatant | (c) F5-444 (1.4 mg/ml) | (d) 4-8-13 (1 mg/ml) |
|---|---|---|---|---|
| anti-human IgG (H+L) | < 7* | $2.3 \times 10^4$ | < 7 | < 7 |
| anti-mouse IgG (H+L) | $1.3 \times 10^4$ | < 10 | $1.4 \times 10^6$ | < 7 |

* Antibody concentration (ng/ml)

## Example 9. Large scale cultivation of transfectoma F5-CHγ1

The following growth media are used for large scale cell cultivation: Medium 1 is a 1:1:1 (v/v) mixture of DMEM containing 4.5 g/l D-glucose (Gibco), Nutrient Mix F12 HAM (Gibco) and RPMI 1640 (Gibco) with the following additions: L-glutamine (300 mg/l, Sigma), sodium pyruvate (73 mg/l, Gibco), glucose (1.7 g/l, Fluka), β-mercaptoethanol (2.3 mg/l, Sigma), ethanolamine (13.3 mg/l, Sigma), $NaHCO_3$ (2.3 g/l, Merck), and gentamycin (10 mg/l, Sigma). Medium 2 has the same composition as Medium 1, but further contains 2% (v/v) Ultroser™ HY (Gibco).

Cells of the transfectoma F5-CHγ1 (example 7) are adapted to growth under serum-free conditions in Medium 2. Permanent stocks of serum-free adapted cells are stored frozen.

For cultivation, cells are passaged in Medium 2 at an initial cell concentration of $4 \times 10^4$ cells/ml at 37°C, 5% $CO_2$, 95% humidity in a Brouwer incubator type 3035. $5 \times 10^8$ cells (91% viability, measured using Trypan Blue™ staining) in 50 ml are used to inoculate an Acusyt-Jr™ automated hollow-fiber cell culture system (Endotronics Inc., Coon Rapids, TN 55433, USA). The culture system is operated according to the manufacturer's instructions using Medium 1 as circulation medium and Medium 2 as cell growth medium. After 7 days growth at 37°C the cycling of Medium 1 is initiated and the first harvest of culture supernatant taken. Cell cultivation is continued for up to 70 days under normal running conditions. The concentration of the antibody in the cell culture supernatant varies between 75 and 350 μg/ml determined using the ELISA of example 7. About 10 to 80 mg of antibody are harvested per day, and the total antibody yield over the production cycle is 1.3 g.

## Example 10. Purification of the chimeric MAb F5-CHγ1

1000 ml of cell culture supernatant kept at -70°C are thawed to room temperature and filtered under sterile conditions through a 0.22 μm filter. The filtrate is pumped at 3 ml/min over a column (2.5 cm x 4 cm) of Sepharose™ CL-4B (Pharmacia) in order to remove any large aggregated material from the solution. The liquid emerging from this column is directly passed through a second column (2.5 cm x 7 cm) of Protein-A agarose (IPA 300, immobilized recombinant Protein-A, Repligen, Cambridge, USA). 100 mM sodium citrate, pH 6.0, is passed through both columns at 3 ml/min until the absorbance at 280 nm (UV-1 monitor, Pharmacia) of the eluate reaches zero and the pH is 6.0 (type 2195 pH/conductivity meter, LKB). The desired antibody bound to the protein-A column is then eluted by pumping 100 mM sodium citrate, pH 4.0, over both columns at 3 ml/min. The eluted antibody is detected by adsorbance at 280 nm and manually collected into sterile 50 ml plastic tubes (Costar). A total volume of 120 ml containing 126 mg antibody (as determined by adsorbance at 280 nm against a known standard antibody preparation) is collected.

The pooled Protein-A eluate is diluted with sterile water until the conductivity (model 4070 conductivity meter, Jenway, Essex, UK) is reduced to 2.3 mS (total volume 500 ml). This solution is passed at 5 ml/min over a pre-packed column of S-Sepharose™ HP (HR 35/100, Pharmacia) equilibrated in 50 mM sodium acetate, pH 5.0. The column is washed with 50 mM sodium acetate at ml/min until the conductivity and pH reach the original equilibrium value and the UV absorbance reaches zero. The bound antibody is eluted by a gradient of 100 % 50 mM sodium acetate, pH 5.0, to 100 % 50 mM sodium acetate and 500 mM NaCl, pH 5.0, over 85 min at a flow rate of 3 ml/min. The antibody elutes starting at 53 min in a major peak containing 110 mg protein and a minor peak containing 10 mg protein (as determined by SDS-PAGE in a 10 % resolving gel under reducing and non-reducing conditions and Coomassie Blue staining). Both peaks are manually collected into two sterile plastic 300 ml bottles, the pH adjusted to pH 7.0 with 1 M Tris base, and the solutions sterile filtered under aseptic conditions through a 0.22 $\mu$m filter (Sterivex-GS™, Millipore) and stored at 4°C. Both the major and the minor peak contain the desired antibody, but the difference in mobility on S-Sepharose™ appears to be the result of differences in the antibody glycosylation.

Example 11 <u>Pharmaceutical preparation for parenteral application</u>

10 mg chimeric antiidiotypic monoclonal antibody F5-CH$\gamma$1 from example 10 are dissolved in 50 ml PBS. The solution is passed through a bacteriological filter, the filtrate divided into 10 equal parts and filled in ampoules under aseptic conditions. The ampoules are preferably stored in the cold, e.g. at 4°C. This pharmaceutical preparation is suitable for injection and is applied as such or in combination with a pharmaceutical preparation containing Alum, a pharmaceutical preparation containing BCG (Bacillus Calmette-Guérin), or a pharmaceutical preparation containing the muramyl peptide MTP-PE.

Sequence listing

SEQ ID NO:1

SEQUENCE TYPE: nucleotide with corresponding protein
SEQUENCE LENGTH: 319 base pairs
MOLECULE TYPE: genomic DNA
ORIGINAL SOURCE ORGANISM: mouse
IMMEDIATE EXPERIMENTAL SOURCE: hybridoma cell line F5-444
NAME OF CELL CLONE: F5-444LPCR39
FEATURES:    from 1 to 24 bp:          primer VK1BACK
                from 7 to 12 bp:          restriction site PvuII
                from 70 to 99 bp:        $CDR_{1L}$
                from 145 to 165 bp:     $CDR_{2L}$
                from 262 to 288 bp:     $CDR_{3L}$
                from 299 to 319 bp:     cDNA to primer VK1FOR
                from 311 to 316 bp:     restriction site BglII
PROPERTIES: light chain variable region of antiidiotypic HLA class II antibody

```
GACATT CAG CTG ACC CAG TCT CCA GCA ATC ATG TCT GCA TCT      42
       Gln Leu Thr Gln Ser Pro Ala Ile Met Ser Ala Ser
                      5               10

CCA GGG GAG AAG GTC ACC ATG ACC TGC AGT GCC AGC TCA AGT      84
Pro Gly Glu Lys Val Thr Met Thr Cys Ser Ala Ser Ser Ser
        15                  20                  25

GTA AGT TAC ATG CAC TGG TAC CAG CAG AAG TCA GGC ACC TCC     126
Val Ser Tyr Met His Trp Tyr Gln Gln Lys Ser Gly Thr Ser
                30              35                      40

CCC AAA AGA TGG ATT TAT GAC ACA TCC AAA CTG ACT TCT GGT     168
Pro Lys Arg Trp Ile Tyr Asp Thr Ser Lys Leu Thr Ser Gly
                    45                  50

GTC CCT GCT CGC TTC AGT GGC AGT GGG TCT GGG ACC TCT TAC     210
Val Pro Ala Arg Phe Ser Gly Ser Gly Ser Gly Thr Ser Tyr
 55                     60                  65

TCT CTC ACA ATC AGC AGC ATG GAG GCT GAA GAT GCT GCC ACT     252
Ser Leu Thr Ile Ser Ser Met Glu Ala Glu Asp Ala Ala Thr
     70                  75                  80

TAT TAC TGC CAG CAG TGG AGT AGT AAC CCG CTC ACG TTC GGT     294
Tyr Tyr Cys Gln Gln Trp Ser Ser Asn Pro Leu Thr Phe Gly
         85                  90                  95

GCT GGC ACC AAG CTG GAG ATC TAAC                            319
Ala Gly Thr Lys Leu Glu Ile
             100
```

SEQ ID NO:2

SEQUENCE TYPE: nucleotide with corresponding protein
SEQUENCE LENGTH: 353 base pairs
MOLECULE TYPE: genomic DNA
ORIGINAL SOURCE ORGANISM: mouse
IMMEDIATE EXPERIMENTAL SOURCE: hybridoma cell line F5-444
NAME OF CELL CLONE: F5-444HPCR3
FEATURES:
| from 1 to 22 bp: | primer VH1BACK |
| from 9 to 14 bp: | restriction site PstI |
| from 90 to 104 bp: | $CDR_{1H}$ |
| from 147 to 194 bp: | $CDR_{2H}$ |
| from 291 to 320 bp: | $CDR_{3H}$ |
| from 320 to 353 bp: | cDNA to primer VH1FOR |
| from 338 to 345 bp: | restriction site BstEII |

PROPERTIES: heavy chain variable region of antiidiotypic HLA class II antibody


```
AGGTGCAA CTG CAG GAG TCA GGA CCT GGC CTG GTG GCG CCC TCA   44
         Leu Gln Glu Ser Gly Pro Gly Leu Val Ala Pro Ser
                          5                   10

CAG AGC CTG TCC ATC ACA TGC ACT GTC TCA GGG TTC TCA ATA     86
Gln Ser Leu Ser Ile Thr Cys Thr Val Ser Gly Phe Ser Ile
        15                  20                  25

ACC GAC TAT GTT GTA AGC TGG ATT CGC CAG CCT CCA GGA AAG    128
Thr Asp Tyr Val Val Ser Trp Ile Arg Gln Pro Pro Gly Lys
            30                  35                  40

GGT CTG GAG TGG CTG GGA GTA ATA TGG GGT GGT GGA AAC ACA    170
Gly Leu Glu Trp Leu Gly Val Ile Trp Gly Gly Gly Asn Thr
                45                  50

TAC TAT AAT TCA GCT CTC AAA TCC AGA CTG AGC ATC AGC AAG    212
Tyr Tyr Asn Ser Ala Leu Lys Ser Arg Leu Ser Ile Ser Lys
 55                  60                  65

GAC AAC TCC AAG AGC CAA GTT TTC TTA AAA ATG AAC AGT CTG    254
Asp Asn Ser Lys Ser Gln Val Phe Leu Lys Met Asn Ser Leu
     70                  75                  80

CAA ACT GAT GAC ACA GCC ATG TAC TAC TGT GCC AAA CAT GAT    296
Gln Thr Asp Asp Thr Ala Met Tyr Tyr Cys Ala Lys His Asp
         85                  90                  95

GAG ATT ACG ACC TAC TTT GAC TAC TGG GGC CAA GGG ACC ACG    338
Glu Ile Thr Thr Tyr Phe Asp Tyr Trp Gly Gln Gly Thr Thr
             100                 105                 110

GTC ACC GTCTCCTCA                                           353
Val Thr
```

## Claims

1.  A chimeric antiidiotypic monoclonal antibody consisting of human constant regions and murine variable regions of an antibody which recognizes antibodies directed against HLA class II antigens, and derivatives thereof which retain the specificity of the antibody from which they are derived.

2. A chimeric antiidiotypic monoclonal antibody according to claim 1 wherein the murine variable regions are the internal image of determinants recognized by an antibody on HLA class II antigens, and derivatives thereof.

3. A chimeric antiidiotypic monoclonal antibody according to claim 1 or 2 wherein the murine variable regions are the internal image of determinants recognized by the monoclonal antibody designated MAb AC1.59 on the allospecificities DR1,4,w6,w8,w9 of HLA class II antigens, and derivatives thereof.

4. The chimeric antiidiotypic monoclonal antibody according to claim 3 with the designation MAb F5-CH$\gamma$1, and derivatives thereof.

5. A chimeric antiidiotypic monoclonal antibody according to any of the claims 1 to 3 wherein the light chain variable regions comprise a polypeptide of the formula
$$\text{FR}_1\text{-CDR}_{1L}\text{-FR}_2\text{-CDR}_{2L}\text{-FR}_3\text{-CDR}_{3L}\text{-FR}_4 \qquad (I)$$
wherein $\text{FR}_1$ is a polypeptide residue comprising 19-23 naturally occuring amino acids, $\text{FR}_2$ is a polypeptide residue comprising 13-17 naturally occurring amino acids, $\text{FR}_3$ is a polypeptide residue comprising 30-34 naturally occurring amino acids, $\text{FR}_4$ is a polypeptide residue comprising 7-11 naturally occurring amino acids, $\text{CDR}_{1L}$ is a polypeptide residue of the amino acid sequence 22 to 31 of SEQ ID NO:1, $\text{CDR}_{2L}$ is a polypeptide residue of the amino acid sequence 47 to 53 of SEQ ID NO:1, and $\text{CDR}_{3L}$ is a polypeptide residue of the amino acid sequence 86 to 94 of SEQ ID NO:1, and wherein the amino acid Cys may be in the oxidized state forming S-S-bridges, and derivatives thereof.

6. A chimeric antiidiotypic monoclonal antibody according to claim 5 wherein the light chain variable regions comprise a polypeptide of the amino acid sequence of SEQ ID NO:1, wherein optionally one or more single amino acids within the amino acid sequences 1 to 21 ($\text{FR}_1$), 32 to 46 ($\text{FR}_2$), 54 to 85 ($\text{FR}_3$), and/or 95 to 103 ($\text{FR}_4$) are replaced by other amino acids or deleted, and wherein the amino acid Cys may be in the oxidized state forming S-S-bridges, and derivatives thereof.

7. A chimeric antiidiotypic monoclonal antibody according to any of the claims 1 to 3 wherein the heavy chain variable regions comprise a polypeptide of the formula
$$\text{FR}_5\text{-CDR}_{1H}\text{-FR}_6\text{-CDR}_{2H}\text{-FR}_7\text{-CDR}_{3H}\text{-FR}_5 \qquad (II)$$
wherein $\text{FR}_5$ is a polypeptide residue comprising 25-29 naturally occurring amino acids, $\text{FR}_6$ is a polypeptide residue comprising 12-16 naturally occurring amino acids, $\text{FR}_7$ is a polypeptide residue comprising 30-34 naturally occurring amino acids, $\text{FR}_5$ is a polypeptide residue comprising 6- 10 naturally occurring amino acids, $\text{CDR}_{1H}$ is a polypepride residue of the amino acid sequence 28 to 32 of SEQ ID NO:2, $\text{CDR}_{2H}$ is a polypeptide residue of the amino acid sequence 47 to 62 of SEQ ID NO:2, and $\text{CDR}_{3H}$ is a polypeptide residue of the amino acid sequence 95 to 104 of SEQ ID NO:2, and wherein the amino acid Cys may be in the oxidized state forming S-S-bridges, and derivatives thereof.

8. A chimeric antiidiotypic monoclonal antibody according to claim 7 wherein the heavy chain variable regions comprise a polypeptide of the amino acid sequence of SEQ ID NO:2, wherein optionally one or more, e.g. 1, 2, 3 or 4, single amino acids within the amino acid sequences 1 to 27 ($\text{FR}_5$), 33 to 46 ($\text{FR}_6$), 63 to 94 ($\text{FR}_7$), and/or 105 to 112 ($\text{FR}_8$) are replaced by other amino acids or deleted, and wherein the amino acid Cys may be in the oxidized state forming S-S-bridges, and derivarives thereof.

9. A chimeric antiidiotypic monoclonal antibody according to any of the claims 1 to 3 or 5 to 8 wherein the light chain region comprises the light chain human constant regions $\kappa$ or $\lambda$, and derivatives thereof.

10. A chimeric antiidiotypic monoclonal antibody according to any of the claims 1 to 3 or 5 to 8 wherein the heavy chain region comprises the heavy chain human constant regions $\gamma$1, $\gamma$2, $\gamma$3 or $\gamma$4, and derivatives thereof.

11. A chimeric antiidiotypic monoclonal antibody according to any of the claims 1 to 10 wherein the light chain variable region comprises the amino acid sequence given in SEQ ID NO:1, wherein the amino acid Cys may be in the oxidized state forming S-S-bridges, the light chain constant region is the light chain human constant region $\kappa$, the heavy chain viable region comprises the amino acid sequence given in SEQ ID NO:2, wherein the amino acid Cys may be in the oxidized state forming S-S-bridges, and the heavy chain constant region is the heavy chain human constant region $\gamma$1.

12. A derivative of a chimeric antiidiotypic monoclonal antibody according to any of the claims 1 to 11 which is an antibody fragment, which is a conjugate of the antibody or fragment with a compound which enhances the antigenicity, with an enzyme, with a fluorescent marker, with a chemiluminescent marker, with a metal chelate, with avidin, or with biotin or the like, or which is a radioactively labelled antibody or fragment.

13. A process for the preparation of a chimeric antiidiotypic monoclonal antibody or a derivative thereof according to any of claims 1 to 12, characterized in that a mammalian cell producing such antibody is multiplied in vitro or in vivo and, when required, the obtained chimeric antiidiotypic monoclonal antibody is isolated and/or converted into a derivative thereof.

14. A recombinant DNA comprising an insert coding for a light chain murine viable region and/or for a heavy chain murine viable region of a chimeric antiidiotypic antibody which recognizes monoclonal antibodies directed against HLA class II antigens.

15. A recombinant DNA according to claim 14 comprising an insert coding for a light chain murine viable region, which originates from genomic DNA of the cell line F5-444 or which is homologous to genomic DNA of said cell line and which codes for an amino acid sequence identical or homologous to the light chain viable region of monoclonal antibody F5-444.

16. A recombinant DNA according to claim 15 comprising an insert coding for the polypeptide of formula
$$FR_1\text{-}CDR_{1L}\text{-}FR_2\text{-}CDR_{2L}\text{-}FR_3\text{-}CDR_{3L}\text{-}FR_4 \qquad (I)$$
wherein $FR_1$, $FR_2$, $FR_3$ and $FR_4$ are murine or human framework regions, $CDR_{1L}$ is a polypeptide residue of the amino acid sequence 22 to 31 of SEQ ID NO:1, $CDR_{2L}$ is a polypeptide residue of the amino acid sequence 47 to 53 of SEQ ID NO:1, and $CDR_{3L}$ is a polypeptide residue of the amino acid sequence 86 to 94 of SEQ ID NO:1.

17. A recombinant DNA according to claim 15 comprising an insert of the DNA sequence 7 to 316 of SEQ ID NO:1, wherein optionally one or more nucleorides are replaced by other nucleorides.

18. A recombinant DNA according to claim 14 comprising an insert coding for a heavy chain murine variable region, which originates from genomic DNA of the cell line F5-444 or which is homologous to genomic DNA of said cell line and which codes for an amino acid sequence identical or homologous to the heavy chain variable region of monoclonal antibody F5-444.

19. A recombinant DNA according to claim 18 comprising an insert coding for the polypeptide of formula
$$FR_5\text{-}CDR_{1H}\text{-}FR_6\text{-}CDR_{2H}\text{-}FR_7\text{-}CDR_{3H}\text{-}FR_5 \qquad (II)$$
wherein $FR_5$, $FR_5$, $FR_7$ and $FR_5$ are murine or human framework regions, $CDR_{1H}$ is a polypeptide residue of the amino acid sequence 28 to 32 of SEQ ID NO:2, $CDR_{2H}$ is a polypeptide residue of the amino acid sequence 47 to 62 of SEQ ID NO:2, and $CDR_{3H}$ is a polypeptide residue of the amino acid sequence 95 to 104 of SEQ ID NO:2.

20. A recombinant DNA according to claim 18 comprising an insert of the DNA sequence 9 to 345 of SEQ ID NO:2, wherein optionally one or more nucleotides are replaced by other nucleotides.

21. A recombinant DNA according to claim 14 comprising an insert coding for a light chain murine variable region of a chimeric antiidiotypic antibody, which recognizes monoclonal antibodies directed against HLA class II antigens, fused to a human constant region $\kappa$ or $\lambda$.

22. A recombinant DNA according to claim 14 comprising an insert coding for a heavy chain murine variable region of a chimeric antiidiotypic antibody, which recognizes monoclonal antibodies directed against HLA class II antigens, fused to a human constant region $\gamma$.

23. A recombinant DNA according to claim 14 which is a hybrid vector comprising an insert coding for a light chain murine variable region of a chimeric antiidiotypic antibodies, which recognizes monoclonal antibodies directed against HLA class II antigens, fused to a human constant region $\kappa$ or $\lambda$ and/or an insert coding for a heavy chain murine variable region of a chimeric antiidiotypic antibody, which recognizes monoclonal antibodies directed against HLA class II antigens, fused to a human constant region $\gamma$, an origin

of replication or an autonomously replicating sequence, one or more dominant marker sequences and, optionally, expression conaol sequences, signal sequences and additional restriction sites.

24. A process for the preparation of a recombinant DNA according to any of claims 14 to 23, comprising the steps of

a) isolating murine DNAs from a suitable hybridoma cell line and selecting the desired DNAs coding for the variable regions of antiidiotypic antibodies with the desired specificity using DNA probes,

b) isolating human DNAs from a genomic library and selecting the desired DNAs coding for the constant regions of antibodies using DNA probes,

c) constructing chimeric mouse/human genes by incorporating the DNA of step a) and b) into appropriate hybrid vectors,

d) transferring the obtained hybrid vectors into a recipient host cell or retrieving the DNA coding for the chimeric mouse/human genes and transferring the unlinked DNA into a recipient host cell,

e) selecting and culturing the transformed host cell, and

f) optionally isolating the desired DNA.

25. A host cell transformed with the recombinant DNA of claim 23.

26. A host cell according to claim 25 which is designated F5-CH$\gamma$1 and was deposited at the European Collection for Animal Cell Cultures (ECACC) on January 31, 1991, under the accession number ECACC 91013172.

27. A process for the preparation of a host cell secreting chimeric antiidiotypic monoclonal antibodies which recognize antibodies directed against HLA class II antiantigens, characterized in that a suitable cell is transformed with one or two hybrid vectors according to claim 23.

28. A method of treating autoimmune diseases and allograft rejections by administering a therapeutically effective amount of a chimeric antiidiotypic monoclonal antibody or of a derivative thereof according to any of claims 1 to 12.

29. A pharmaceutical composition for treating autoimmune diseases and allograft rejections comprising a therapeutically effective amount of a chimeric antiidiotypic monoclonal antibody and/or a derivative thereof according to any of claims 1 to 12, and a pharmaceutically acceptable carrier.

30. Use of a chimeric antiidiotypic monoclonal antibody and/or of a derivative thereof according to any of claims 1 to 12 for the qualitative and/or quantitative determination of antibodies directed against HLA class II antigens.

31. A test kit for the qualitative and/or quantitative determination of antibodies directed against HLA class II antigens comprising chimeric antiidiotypic monoclonal antibodies and/or a derivative thereof according to any of claims 1 to 12 and, optionally, other polyclonal or monoclonal antibodies and/or adjuncts.

**Claims for the following Contracting States : ES, GR**

1. A process for the preparation of a chimeric antiidiotypic monoclonal antibody consisting of human constant regions and murine variable regions of an antibody which recognizes antibodies directed against HLA class II antigens, or a derivative thereof which retains the specificity of the antibody from which it is derived, characterized in that a mammalian cell producing such antibody is multiplied in vitro or in vivo and, when required, the obtained chimeric antiidiotypic monoclonal antibody is isolated and/or converted into a derivative thereof.

2. A process according to claim 1, characterized in that the murine variable regions of the chimeric antiidiotypic monoclonal antibodies are the internal image of determinants recognized by an antibody on HLA class II antigens.

3. A process according to claim 1 or 2, characterized in that the murine variable regions of the chimeric antiidiotypic monoclonal antibodies are the internal image of determinants recognized by the monoclonal antibody designated MAb AC1.59 on the allospecificities DR1,4,w6,w8,w9 of HLA class II antigens.

4. A process according to claim 3, characterized in that the chimeric antiidiotypic monoclonal antibody is designated MAb F5-CHγ1.

5. A process according to any of the claims 1 to claim 3, characterized in that the the light chain variable regions of the chimeric antiidiotypic monoclonal antibodies comprise a polypeptide of the formula

$$FR_1\text{-}CDR_{1L}\text{-}FR_2\text{-}CDR_{2L}\text{-}FR_3\text{-}CDR_{3L}\text{-}FR_4 \qquad (I)$$

wherein $FR_1$ is a polypeptide residue comprising 19-23 naturally occurring amino acids, $FR_2$ is a polypeptide residue comprising 13-17 naturally occurring amino acids, $FR_3$ is a polypeptide residue comprising 30-34 natwally occurring amino acids, $FR_4$ is a polypeptide residue comprising 7-11 naturally occurring amino acids, $CDR_{1L}$ is a polypeptide residue of the amino acid sequence 22 to 31 of SEQ ID NO: 1, $CDR_{2L}$ is a polypeptide residue of the amino acid sequence 47 to 53 of SEQ ID NO: 1, and $CDR_{3L}$ is a polypeptide residue of the amino acid sequence 86 to 94 of SEQ ID NO: 1, and wherein the amino acid Cys may be in the oxidized state forming S-S-bridges.

6. A process according to claim 5, characterized in that the the light chain variable regions of the chimeric antiidiotypic monoclonal antibodies comprise a polypeptide of the amino acid sequence of SEQ ID NO: 1, wherein optionally one or more single amino acids within the amino acid sequences 1 to 21 ($FR_1$), 32 to 46 ($FR_2$), 54 to 85 ($FR_3$), and/or 95 to 103 ($FR_4$) are replaced by other amino acids or deleted, and wherein the amino acid Cys may be in the oxidized state forming S-S-bridges.

7. A process according to any of the claims 1 to claim 3, characterized in that the the heavy chain viable regions of the chimeric antiidiotypic monoclonal antibodies comprise a polypeptide of the formula

$$FR_5\text{-}CDR_{1H}\text{-}FR_6\text{-}CDR_{2H}\text{-}FR_7\text{-}CDR_{3H}\text{-}FR_5 \qquad (II)$$

wherein $FR_5$ is a polypeptide residue comprising 25-29 naturally occurring amino acids, $FR_6$ is a polypeptide residue comprising 12-16 naturally occurring amino acids, $FR_7$ is a polypeptide residue comprising 30-34 naturally occurring amino acids, $FR_5$ is a polypeptide residue comprising 6-10 naturally occurring amino acids, $CDR_{1H}$ is a polypeptide residue of the amino acid sequence 28 to 32 of SEQ ID NO:2, $CDR_{2H}$ is a polypeptide residue of the amino acid sequence 47 to 62 of SEQ ID NO:2, and $CDR_{3H}$ is a polypeptide residue of the amino acid sequence 95 to 104 of SEQ ID NO:2, and wherein the amino acid Cys may be in the oxidized state forming S-S-bridges.

8. A process according to claim 7, characterized in that the the heavy chain viable regions of the chimeric antiidiotypic monoclonal antibodies comprise a polypeptide of the amino acid sequence of SEQ ID NO:2, wherein optionally one or more, e.g. 1, 2, 3 or 4, single amino acids within the amino acid sequences 1 to 27 ($FR_5$), 33 to 46 ($FR_5$), 63 to 94 ($FR_7$), and/or 105 to 112 ($FR_5$) are replaced by other amino acids or deleted, and wherein the amino acid Cys may be in the oxidized state forming S-S-bridges.

9. A process according to any of the claims 1 to 3 or 5 to 8, characterized in that wherein the light chain region of the chimeric antiidiotypic monoclonal antibodies comprises the light chain human constant regions κ or λ.

10. A process according to any of the claims 1 to 3 or 5 to 8, characterized in that wherein the heavy chain region of the chimeric antiidiotypic monoclonal antibodies comprises the heavy chain human constant regions γ1, γ2, γ3 or γ4.

11. A process according to any of the claims 1 to 10, characterized in that the light chain variable region of the chimeric antiidiotypic monoclonal antibodies comprises the amino acid sequence given in SEQ ID NO:1, wherein the amino acid Cys may be in the oxidized state forming S-S-bridges, the light chain constant region is the light chain human constant region κ, the heavy chain variable region comprises the amino acid sequence given in SEQ ID NO:2, wherein the amino acid Cys may be in the oxidized state forming S-S-bridges, and the heavy chain constant region is the heavy chain human constant region γ1.

12. A process according to any of the claims 1 to 11 for the prepararion of a derivative of a chimeric antiitiotypic monoclonal antibody, which is an antibody fragment, which is a conjugate of the antibody or fragment with a compound which enhances the antigenicity, with an enzyme, with a fluorescent marker, with a chemiluminescent marker, with a metal chelate, with avidin, or with biotin or the like, or which is a radioactively labelled antibody or fragment.

13. A process for the preparation of a recombinant DNA comprising an insert coding for a light chain murine variable region and/or for a heavy chain murine variable region of a chimeric antiidiotypic antibody which recognizes monoclonal antibodies directed against HLA class II antigens which comprises the steps of

a) isolating murine DNAs from a suitable hybridoma cell line and selecting the desired DNAs coding for the variable regions of antiidiotypic antibodies with the desired specificity using DNA probes,

b) isolating human DNAs from a genomic library and selecting the desired DNAs coding for the constant regions of antibodies using DNA probes,

c) constructing chimeric mouse/human genes by incorporating the DNA of step a) and b) into appropriate hybrid vectors,

d) transferring the obtained hybrid vectors into a recipient host cell or retrieving the DNA coding for the chimeric mouse/human genes and transferring the unlinked DNA into a recipient host cell,

e) selecting and culturing the transformed host cell, and

f) optionally isolating the desired DNA.

14. A process according to claim 13, characterized in that the recombinant DNA comprises an insert coding for a light chain murine variable region, which originates from genomic DNA of the cell line F5-444 or which is homologous to genomic DNA of said cell line and which codes for an amino acid sequence identical or homologous to the light chain variable region of monoclonal antibody F5-444.

15. A process according to claim 14, characterized in that the recombinant DNA comprises an insert coding for the polypeptide of formula

$$FR_1\text{-}CDR_{1L}\text{-}FR_2\text{-}CDR_{2L}\text{-}FR_3\text{-}CDR_{3L}\text{-}FR_4 \qquad (I)$$

wherein $FR_1$, $FR_2$, $FR_3$ and $FR_4$ are murine or human framework regions, $CDR_{1L}$ is a polypeptide residue of the amino acid sequence 22 to 31 of SEQ ID NO:1, $CDR_{2L}$ is a polypeptide residue of the amino acid sequence 47 to 53 of SEQ ID NO:1, and $CDR_{3L}$ is a polypeptide residue of the amino acid sequence 86 to 94 of SEQ ID NO:1.

16. A process according to claim 14, characterized in that the recombinant DNA comprises an insert of the DNA sequence 7 to 316 of SEQ ID NO:1, wherein optionally one or more nucleotides are replaced by other nucleotides.

17. A process according to claim 13, characterized in that the recombinant DNA comprises an insert coding for a heavy chain murine variable region, which originates from genomic DNA of the cell line F5-444 or which is homologous to genomic DNA of said cell line and which codes for an amino acid sequence identical or homologous to the heavy chain variable region of monoclonal antibody F5-444.

18. A process according to claim 17, characterized in that the recombinant DNA comprises an insert coding for the polypeptide of formula

$$FR_5\text{-}CDR_{1H}\text{-}FR_6\text{-}CDR_{2H}\text{-}FR_7\text{-}CDR_{3H}\text{-}FR_5 \qquad (II)$$

wherein $FR_5$, $FR_5$, $FR_7$ and $FR_5$ are murine or human framework regions, $CDR_{1H}$ is a polypeptide residue of the amino acid sequence 28 to 32 of SEQ ID NO:2, $CDR_{2H}$ is a polypeptide residue of the amino acid sequence 47 to 62 of SEQ ID NO:2, and $CDR_{3H}$ is a polypeptide residue of the amino acid sequence 95 to 104 of SEQ ID NO:2.

19. A process according to claim 17, characterized in that the recombinant DNA comprises an insert of the DNA sequence 9 to 345 of SEQ ID NO:2, wherein optionally one or more nucleotides are replaced by other nucleotides.

20. A process according to claim 13, characterized in that the recombinant DNA comprises an insert coding for a light chain murine variable region of a chimeric antiidiotypic antibody, which recognizes monoclonal antibodies directed against HLA class II antigens, fused to a human constant region κ or λ.

21. A process according to claim 13, characterized in that the recombinant DNA comprises an insert coding for a heavy chain murine variable region of a chimeric antiidiotypic antibody, which recognizes monoclonal antibodies directed against HLA class II antigens, fused to a human constant region γ.

22. A process according to claim 13 for the preparation of a recombinant DNA which is a hybrid vector comprising an insert coding for a light chain murine variable region of a chimeric antiidiotypic antibody, which recognizes monoclonal antibodies directed against HLA class II antigens, fused to a human constant region κ or λ and/or an insert coding for a heavy chain murine variable region of a chimeric antiidiotypic antibody, which recognizes monoclonal antibodies directed against HLA class II antigens, fused to a human constant region y, an origin of replication or an autonomously replicating sequence, one or more dominant marker sequences and, optionally, expression control sequences, signal sequences and additional restriction sites.

23. A process for the preparation of a host cell secreting chimeric antiidiotypic monoclonal antibodies which recognize antibodies directed against HLA class II antigens, characterized in that a suitable cell is transformed with one or two hybrid vectors prepared by a process according to claim 22.

24. A process for the preparation of a host cell according to claim 23, characterized in that the host cell is designated F5-CHγ1 and was deposited at the European Collection for Animal Cell Cultures (ECACC) on January 31, 1991, under the accession number ECACC 91013172.

25. Use of a chimeric antiidiotypic monoclonal antibody and/or of a derivative thereof prepared by a process according to any of claims 1 to 12 for the qualitative and/or quantitative determination of antibodies directed against HLA class II antigens.

26. A test kit for the qualitative and/or quantitative determination of antibodies directed against HLA class II antigens comprising chimeric antiidiotypic monoclonal antibodies and/or a derivative thereof prepared by a process according to any of claims 1 to 12 and, optionally, other polyclonal or monoclonal antibodies and/or adjuncts.